# EUROPEAN PATENT APPLICATION

(11) **EP 4 667 455 A1**
(43) Date of publication of application: **24.12.2025**
(21) Application number: 24755865.3
(22) Date of filing: 16.01.2024
(51) Int. Cl.: C07D 207/27, C07B 53/00, B01J 31/24, C07F 17/02, C07F 19/00

(54) **ASYMMETRIC CATALYTIC HYDROGENATION SYNTHESIS METHOD FOR BRIVARACETAM INTERMEDIATE**

(30) Priority: 17.02.2023 CN 202310129899
(71) Applicant: Yangzhou Aurisco Pharmaceutical Co., Ltd, Yangzhou, Jiangsu 225100 (CN); Shanghai Jiao Tong University, Shanghai 200240 (CN)
(72) Inventor: ZHANG, Wanbin, Shanghai 200240 (CN); WANG, Guoping, Yangzhou, Jiangsu 225100 (CN); LIU, Delong, Shanghai 200240 (CN); WANG, Guangjie, Yangzhou, Jiangsu 225100 (CN); DING, Zhengdong, Shanghai 200240 (CN); LU, Yufei, Shanghai 200240 (CN); YU, Zhenpeng, Yangzhou, Jiangsu 225100 (CN)
(74) Representative: Dehns
(86) International application number: PCT/CN2024/072430
(87) International publication number: WO 2024/169490

(57) **Abstract**

The present invention relates to an asymmetric catalytic hydrogenation synthesis method for a brivaracetam intermediate. The synthesis method comprises the step of: in a hydrogen atmosphere, in a solvent, a compound represented by formula I is subjected to asymmetric hydrogenation reduction in the presence of an alkali and a catalyst which is formed by a planar chiral metallocene ligand and a ruthenium salt to form a compound as represented by formula II, wherein the reaction formula is shown below. In the present invention, the catalyst used in the asymmetric catalytic hydrogenation synthesis method has low cost, the conversion rate of raw materials is high, and the DR value of the obtained final product can reach 99: 1 or above; and the present invention is suitable for industrial production

## Description

This application claims priority to Chinese patent application number CN202110185444.7, filed on February 10, 2021. This application cites the full text of the aforementioned Chinese patent application.

### TECHNICAL FIELD

The present invention relates to the field of synthesis of organic compound, specifically to the asymmetric catalytic hydrogenation synthesis method of (2S)-2-[(4R)-2-oxo-4-propyl-1-pyrrolidinyl] butyric acid.

### BACKGROUND OF THE INVENTION

Brivaracetam, also known as (2S)-2-[(4R)-2-oxo-4-propyl-1-pyrrolidinyl] butanamide, has the following structure:

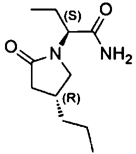

Brivaracetam is a third-generation antiepileptic drug developed by Belgian company UCB. It was approved by EMEA and FDA for market launch in January and February 2016, respectively, for the treatment of partial seizures in adult and adolescent epilepsy patients aged 16 and above, with or without secondary systemic seizures as adjuvant therapy.

The originator company UCB disclosed in WO01/62726 a method for preparing (2S)-2-[(4R)-2-oxo-4-propyl-1-pyrrolidinyl] butanamide. The method used Pd/C as a catalyst to hydrogenate compound 374 in the presence of NH₄COOH, thereby obtaining two enantiomers 158 and 159, with a ratio of nearly 1:1.

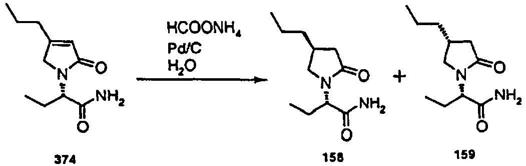

WO2017/076738A1 has improved the scheme described in WO01/62726, specifically by using Pt/C as a catalyst to catalyze the hydrogenation of the compound shown in formula (III) in the presence of formic acid or citric acid to generate the compounds shown in formulas (Ia) and (Ib). The reaction formula was as follows. The chiral purity of the target compound in the crude product has been improved, and the DE value of the obtained product was about 61%.

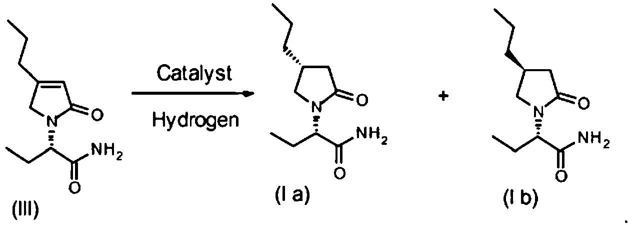

Both of the above methods for preparing brivaracetam require the use of chromatographic columns to separate enantiomers, making it difficult to achieve mass production. Guangzhou Saifeng Pharmaceutical Technology Co., Ltd. attempted to prepare brivaracetam with high optical purity through asymmetric reduction to address this issue in CN104892483A. It disclosed a method for preparing 2-oxo-1-pyrrolidine chiral derivatives, which used transition metal as a catalyst and silane as a hydrogen source in the presence of phosphorus ligand at low temperature to obtain highly optically pure brivaracetam. However, the chiral ligand used in this method has complex structure and is difficult to obtain in large quantities and expensive in cost, which seriously restricted its industrial application. In addition, chiral induced catalysis often requires strict reaction conditions, and serious amplification effect often occurs in production, further posing difficulties for its subsequent application.

CN107513031A disclosed a method for preparing chiral derivatives of 2-oxo-1-pyrrolidine, which involves using catalysts such as palladium carbon, palladium alumina, palladium silica, palladium barium carbonate, palladium calcium carbonate, palladium hydroxide on carbon, nail carbon, Raney nickel, platinum dioxide, and rhodium carbon to catalyze the compound shown in formula VI to generate the compound shown in formula I ( 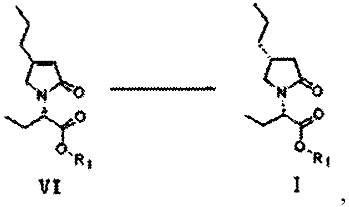 wherein, R₁ is hydrogen or alkyl). In this prior art, (2S)-2-(4-propyl-1,5-dihydropyrrol-2-one) butyric acid was used as the substrate for catalytic hydrogenation to obtain (2S)-2-[(4R)-2-oxo-4-propyl-1-pyrrolidinyl] butyric acid. The product obtained by recrystallization twice has a yield of 80%, and a de value of 95%, and no conversion rate was disclosed. WO2019/157856A1 disclosed a method for preparing lactam intermediates with high optical purity, which involves hydrogenation and reduction of compound C in the presence of heavy metal catalyst (palladium on carbon, palladium, platinum on carbon, platinum, ruthenium on carbon, rhodium on carbon, palladium alumina, palladium silica, palladium barium carbonate, palladium calcium carbonate, palladium hydroxide on carbon, palladium dioxide) and chiral inducers (formic acid, citric acid, malonic acid, succinic acid, hydrochloric acid, hydrobromic acid, etc.) to obtain compound D: ( 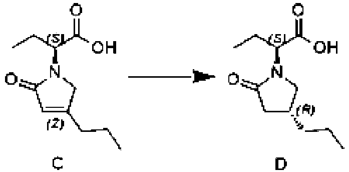 ). The crystallized product was obtained with a yield of 77-96%, and a de value of 99.0-99.6%, and no conversion rate was disclosed. The inventors of the present application found in actual large-scale production that although the de value of the product obtained by using heavy metal catalyst (such as palladium on carbon) to catalyze the hydrogenation of (2S)-2-(4-propyl-1,5-dihydropyrrol-2-one) butyric acid was high, the conversion rate of the substrate was not high enough, and there are unreacted raw materials in the target product. Moreover, it was difficult to remove the raw materials from the target product either through recrystallization or column chromatography, resulting in high purification cost and low quality of the product.

In summary, the existing schemes for obtaining brivaracetam or the intermediates thereof through catalytic hydrogenation are not ideal. Therefore, there is an urgent demand in the field for a cost-effective, simple-to-operate preparation process of Brivaracetam intermediates, which is capable of delivering highly optically pure product.

### SUMMARY OF THE INVENTION

For the deficiencies in the preparation process of brivaracetam and the intermediates thereof in prior arts, one aspect of the present invention is to provide an asymmetric catalytic hydrogenation synthesis method for the compound shown in formula II, the synthesis method comprises: in a hydrogen atmosphere, in a solvent, the compound represented by formula I is subjected to asymmetric hydrogenation reduction in the presence of an alkali and a catalyst which is formed by a planar chiral metallocene ligand and a ruthenium salt, to form a compound as represented by formula II, wherein the reaction formula is shown below: 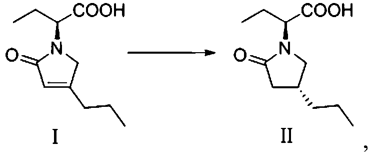
the planar chiral metallocene ligand is selected from the compound represented by the following formulas III to VI: 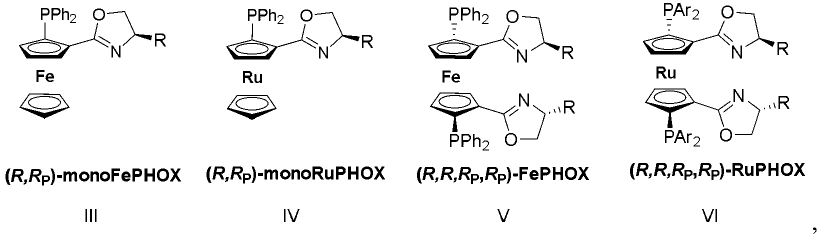 in formulas III-VI, R is selected from H, linear or branched alkyl containing 1-8 carbon atoms, cycloalkyl containing 1-8 carbon atoms, and substituted or unsubstituted aryl or benzyl containing 6-10 carbon atoms, in formula VI, Ar is a substituted or unsubstituted aryl containing 6-10 carbon atoms, and "substituted" means that one or more hydrogen atoms on the aryl or benzyl can be optionally substituted by alkyl, haloalkyl, alkoxy, cycloalkyl, aryl, benzyl or halogen,
the ruthenium salt is selected from the group consisting of tris (triphenylphosphine) ruthenium chloride, dichloro (p-methylisopropylphenyl) ruthenium (II) dimer, phenylruthenium (II) chloride dimer, bis(2,2'-bipyridyl) ruthenium dichloride, dichloro(1,5-cyclooctadiene)ruthenium (II), benzylidene-bis(tricyclohexylphosphine)dichlororuthenium (II), p-isopropylphenyl triphenylphosphine ruthenium chloride, bis (triphenylphosphine) cyclopentadienyl ruthenium chloride (II), chloro (indenyl) bis (triphenylphosphine) ruthenium (II), pentamethyl cyclopentadienyl bis (triphenylphosphine) ruthenium chloride (II), dichlorodicarbonyl bis (triphenylphosphine) ruthenium, dichlorobis [(2-propylphosphine) ethylamine] ruthenium (II), carbonyl chlorohydrotris (triphenylphosphine) ruthenium, and the combinations thereof.

In another preferred embodiment of the present invention, "substituted" means that one or more hydrogen atoms on the aryl or benzyl can be optionally substituted by alkyl containing 1-8 carbon atoms, halogenated alkyl containing 1-8 carbon atoms, alkoxy containing 1-8 carbon atoms, cycloalkyl containing 1-8 carbon atoms, aryl containing 6-10 carbon atoms, benzyl, or halogen.

In another preferred embodiment of the present invention, in the above formulas III-VI, R is selected from the group consisting of methyl, ethyl, isopropyl, *tert-butyl,* substituted or unsubstituted aryl, or benzyl containing 6-10 carbon atoms. In another preferred embodiment of the present invention, in the above formulas III-VI, R is selected from the group consisting of isopropyl and *tert-butyl.* In another preferred embodiment of the present invention, Ar is phenyl, or phenyl substituted with methyl or trifluoromethyl.

In another preferred embodiment of the present invention, the planar chiral metallocene ligand is selected from compounds represented by Formula IV or VI. In another preferred embodiment of the present invention, the ruthenium salt is selected from the group consisting of tris (triphenylphosphine) ruthenium chloride, dichloro (p-methylisopropylphenyl) ruthenium (II) dimer, and phenylruthenium (II) chloride dimer.

In another preferred embodiment of the present invention, the catalyst is selected from the compounds shown in the following structure:

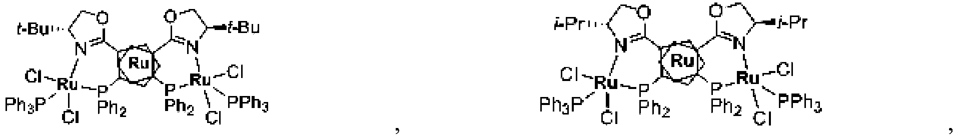

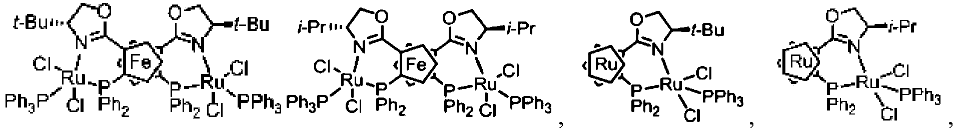

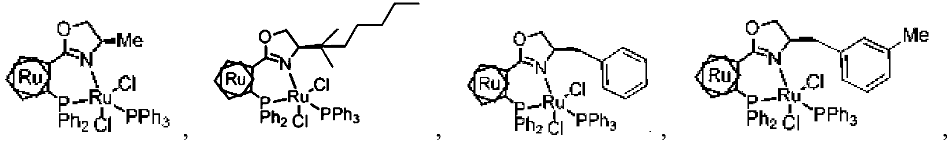

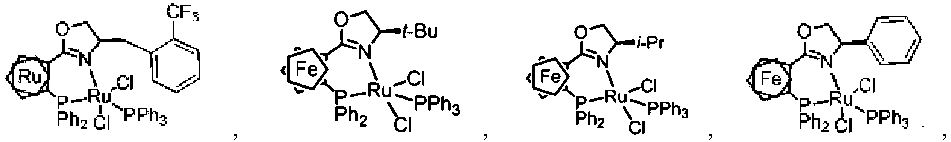

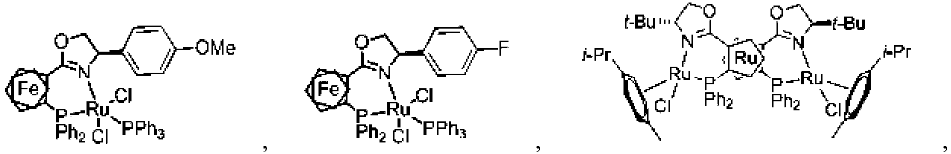

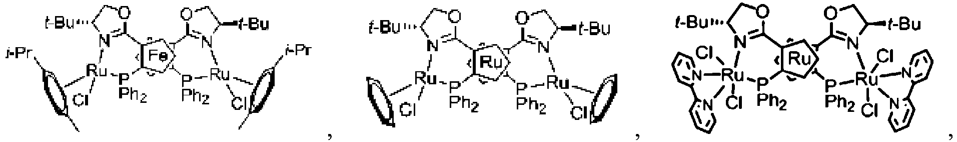

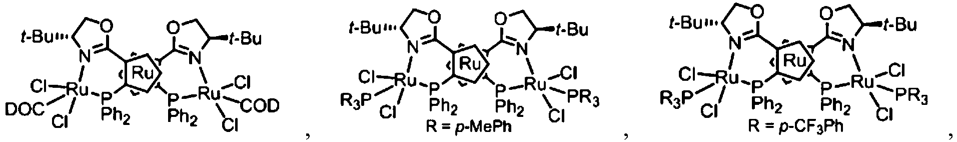

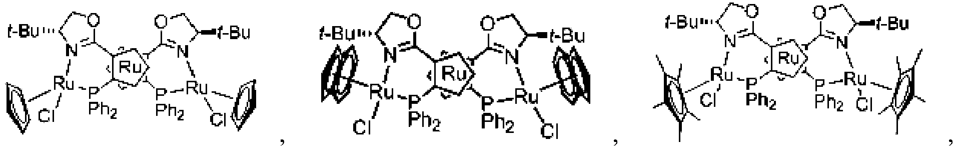

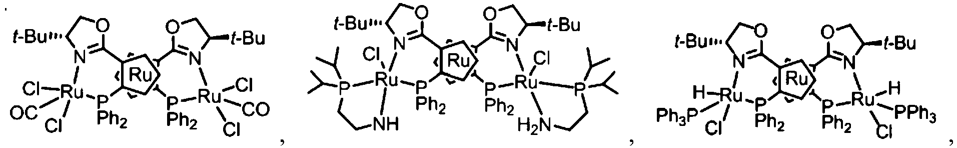

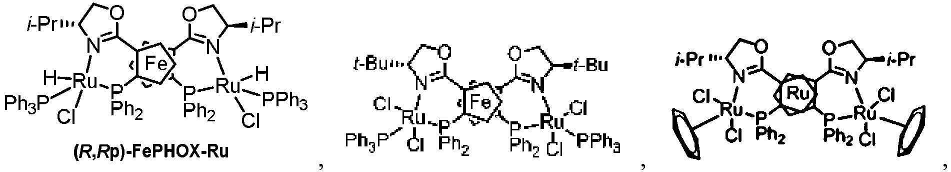

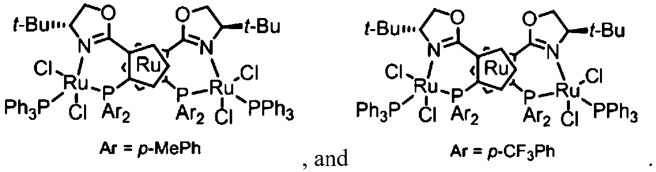

In another more preferred embodiment of the present invention, the catalyst is selected from the compounds shown in the following structure: 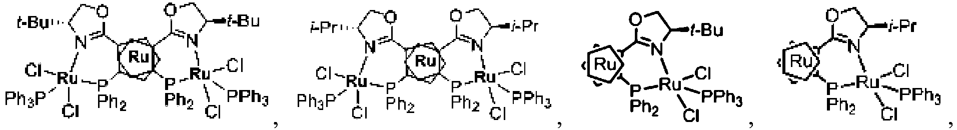 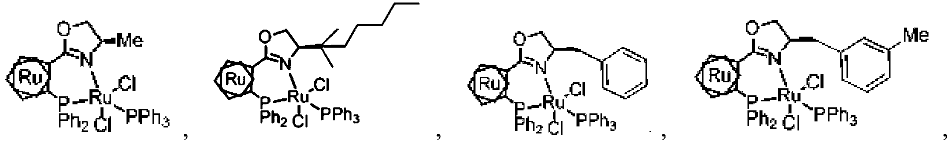 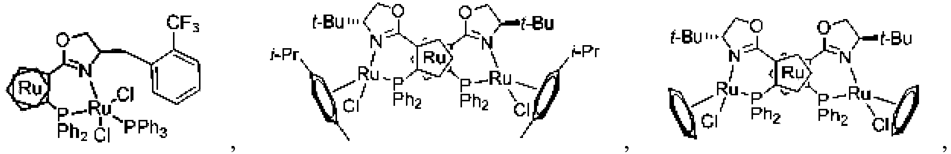 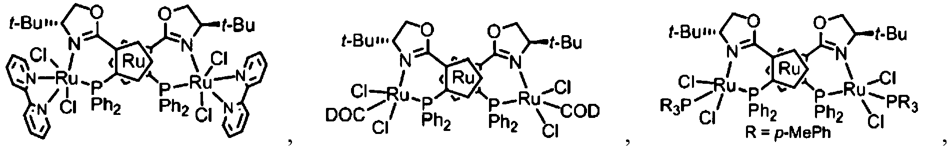 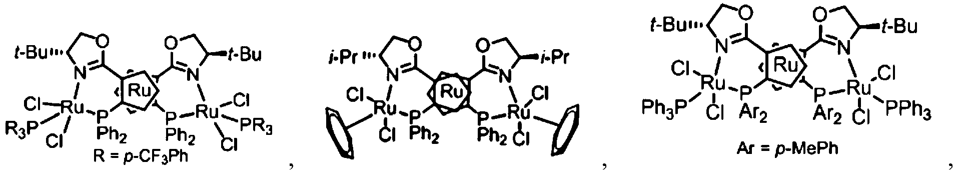 and

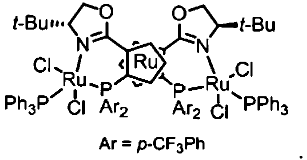

In another more preferred embodiment of the present invention, the catalyst is selected from the compound shown in the following structure:

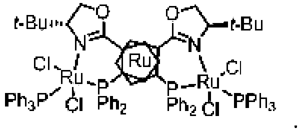

In another preferred embodiment of the present invention, the solvent is selected from the group consisting of methanol, ethanol, isopropanol, propanol, butanol, isobutanol, acetone, 1,4-dioxane, tetrahydrofuran, dichloromethane, acetonitrile, toluene, xylene, and the combinations thereof. In another more preferred embodiment of the present invention, the solvent is selected from the group consisting of methanol, ethanol, and isopropanol.

In another preferred embodiment of the present invention, the alkali is selected from the group consisting of LiOH, NaOH, KOH, Li₂CO₃, Na₂CO₃, K₂CO₃, Cs₂CO₃, LiHCO₃, NaHCO₃, KHCO₃, CsHCO₃, Na₃PO₄, Na₂HPO₄, NaH₂PO₄, triethylamine, pyridine, 2,6-methylpyridine, 1,8-diazabicyclo[5,4,0]-7-undecene, 1,4-diaza[2.2.2]bicyclooctane, and the combinations thereof. In another more preferred embodiment of the present invention, the alkali is selected from NaOH and/or KOH.

In another preferred embodiment of the present invention, the molar ratio of the compound represented by formula I to the alkali is 1:0.1-3. In another more preferred embodiment of the present invention, the molar ratio of the compound represented by formula I to the alkali is 1:0.8~1.5. In another more preferred embodiment of the present invention, the molar ratio of the compound represented by formula I to the alkali is 1:1.

In another preferred embodiment of the present invention, the reaction temperature of the asymmetric catalytic hydrogenation synthesis method is -20-60 °C. In another more preferred embodiment of the present invention, the reaction temperature of the asymmetric catalytic hydrogenation synthesis method is 15-50 °C, for example, 25-35 °C.

In another preferred embodiment of the present invention, the hydrogen pressure of the asymmetric catalytic hydrogenation synthesis method is 1 to 80 bar, more preferably 40 to 60 bar, such as 40 bar, 45 bar, 50 bar, 55 bar, or 60 bar.

In another preferred embodiment of the present invention, the molar ratio of the substrate to the catalyst in the asymmetric catalytic hydrogenation synthesis method is 10000:1-10:1, more preferably 3000:1-100:1.

In another preferred embodiment of the present invention, the method for generating a catalyst by reacting a planar chiral metallocene ligand with a ruthenium salt comprises the steps of: reacting a planar chiral metallocene ligand with a ruthenium salt in a solvent at 60-120 °C to generate a catalyst. In another more preferred embodiment of the present invention, in the method for generating a catalyst by reacting a planar chiral metallocene ligand with a ruthenium salt, the reaction temperature is 70-100 °C.

In another preferred embodiment of the present invention, the solvent used for the reaction of a planar chiral metallocene ligand with a ruthenium salt to generate catalysts is selected from the group consisting of methanol, ethanol, isopropanol, propanol, butanol, isobutanol, acetone, 1,4-dioxane, tetrahydrofuran, dichloromethane, acetonitrile, toluene, xylene, and the combinations thereof.

In another preferred embodiment of the present invention, the asymmetric catalytic hydrogenation synthesis method of the compound shown in formula II comprises the following steps:
(1) reacting a planar chiral ruthenocene ligand with a ruthenium salt in a solvent, and after the reaction is completed, treating and separating the reaction solution to obtain a catalyst;
(2) adding the compound shown in formula I, the catalyst obtained in step (1), an alkali, and a solvent into a reaction vessel, and conducting the asymmetric hydrogenation reduction under a hydrogen atmosphere to form the compound shown in formula II,
the solvents used in step (1) and step(2) may be the same or different.

In another preferred embodiment of the present invention, the asymmetric catalytic hydrogenation synthesis method of the compound shown in formula II comprises the following steps:
(1) reacting a planar chiral ferrocene ligand with a ruthenium salt in a solvent to form a catalyst, obtaining a reaction solution containing the catalyst,
(2) adding the compound of formula I, an alkali, and a solvent to the reaction solution containing the catalyst in step (1), in a hydrogen atmosphere, the compound shown in formula I undergoing the asymmetric hydrogenation reduction to form the compound shown in formula II,
the solvents used in step (1) and step (2) are preferably the same.

Another aspect of the present invention provides a synthesis method for brivaracetam, the synthesis method comprises the following steps:
(1) subjecting the compound shown in formula II obtained by the asymmetric catalytic hydrogenation synthesis method described above to esterification reaction to obtain the compound shown in formula II-1,
(2) subjecting the compound shown in II-1 to an amination reaction to obtain brivaracetam, and the reaction formula is as follows: 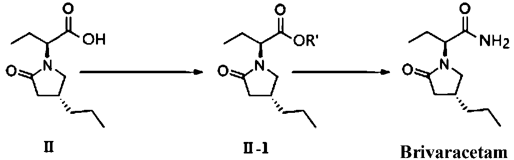
in formula II-1, R' is selected from aliphatic or aromatic hydrocarbon groups.

In another preferred embodiment, the esterification reaction of step (1) is carried out in methanol or ethanol solvent in the presence of thionyl chloride. The amount of raw materials and reaction temperature follow the conventional amount and temperature for this type of reaction. For example, adding thionyl chloride dropwise to methanol at -10-0 °C, stirring at -15~-5 °C for 0.5~1.5 hours, for the compound shown in formula II, stirring at -15~-5 °C for 0.5-1.5 hours, heating to 0-10 °C, stirring for 1.5-3 hours, and detecting the reaction until completed by TLC.

In another preferred embodiment, the amination reaction of step (2) is carried out in a mixed solvent of ammonia water with a mass concentration of 15-28% and alcohol, under the condition of introduced ammonia gas at -10~5 °C, wherein the alcohol is methanol and/or ethanol.

Another aspect of the present invention provides a use of the compound as shown in the following structure for asymmetric catalytic hydrogenation of 1,5-dihydropyrrol-2-one and the derivatives thereof, wherein 1,5-dihydropyrrol-2-one and the derivatives thereof are subjected to hydrogenation reduction to obtain 2-pyrrolidone and the derivatives thereof,

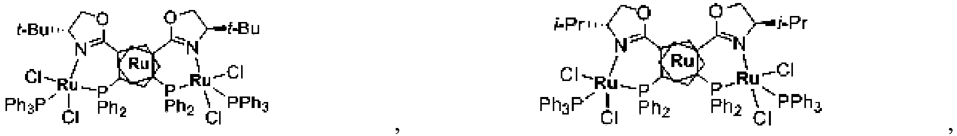

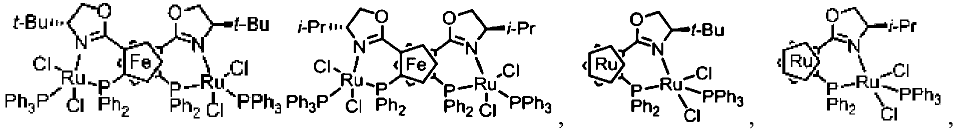

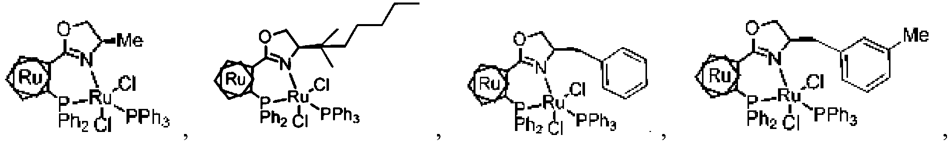

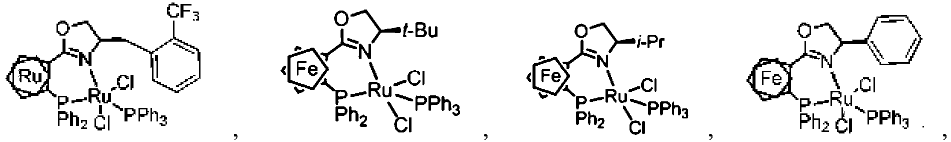

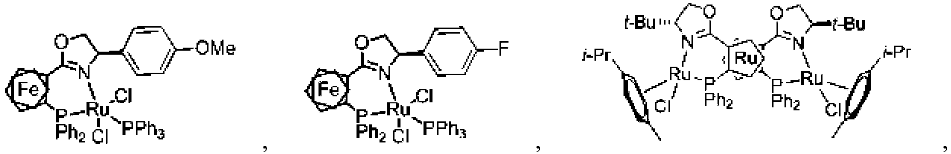

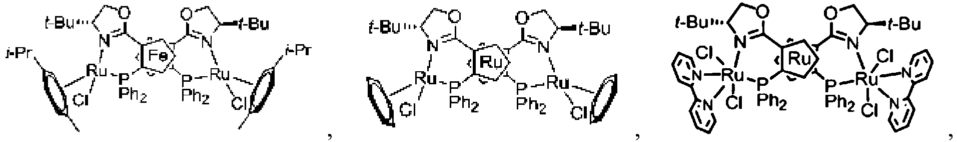

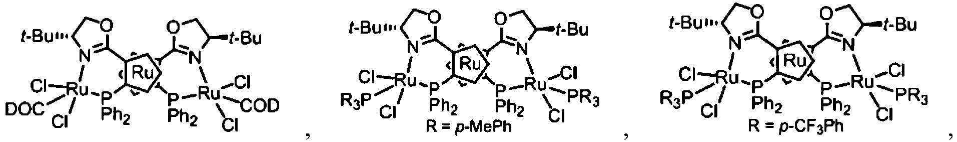

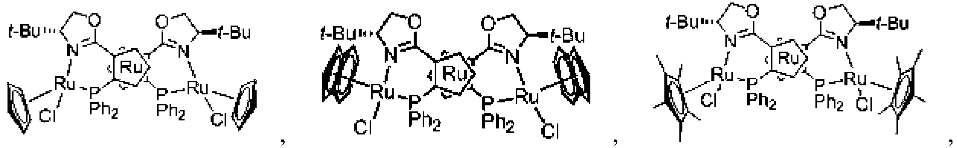

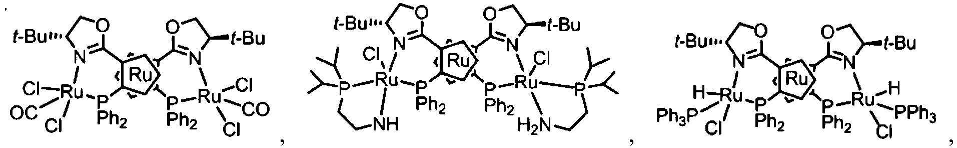

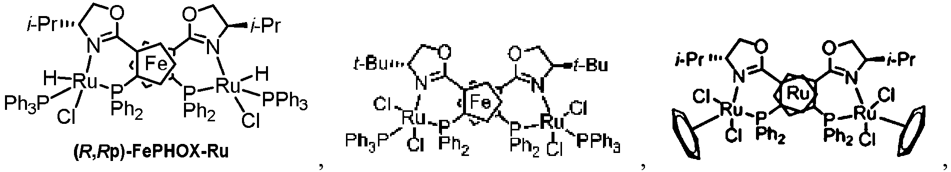

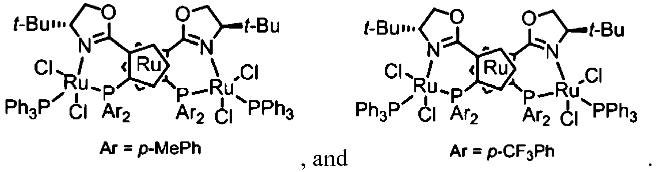

Another aspect of the present invention provides the use of a compound as shown in the following structure for asymmetric catalytic hydrogenation of 1,5-dihydropyrrol-2-one and the derivatives thereof to obtain 2-pyrrolidone and the derivatives thereof,

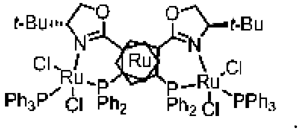

Further another aspect of the present invention provides a catalyst for asymmetric hydrogenation reduction, which has the following structure:

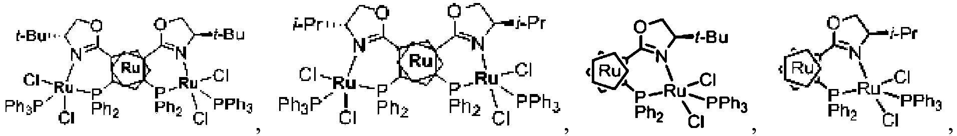

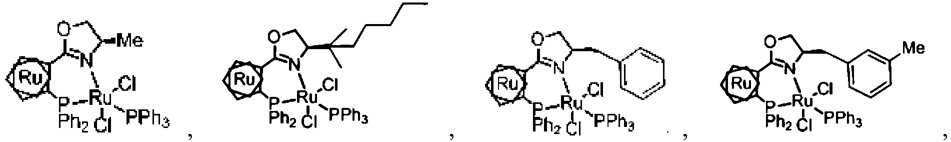

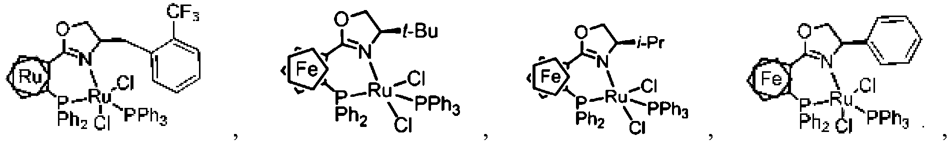

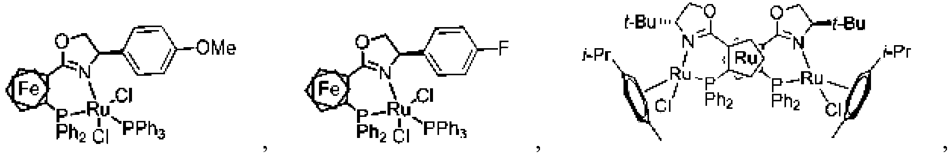

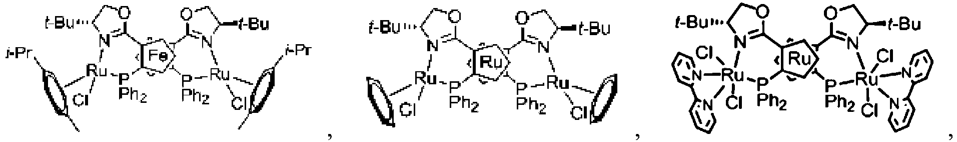

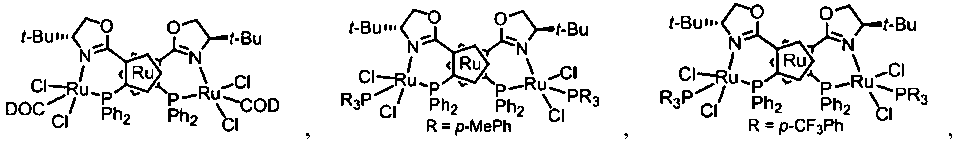

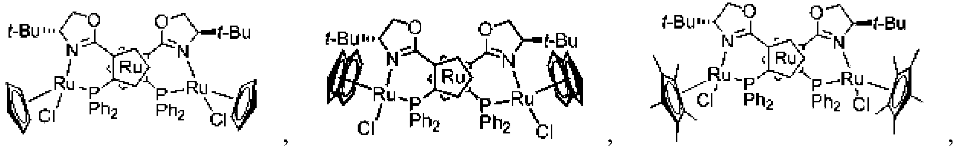

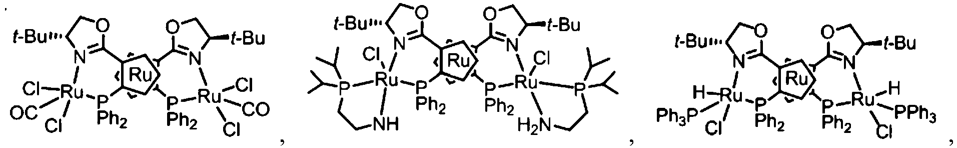

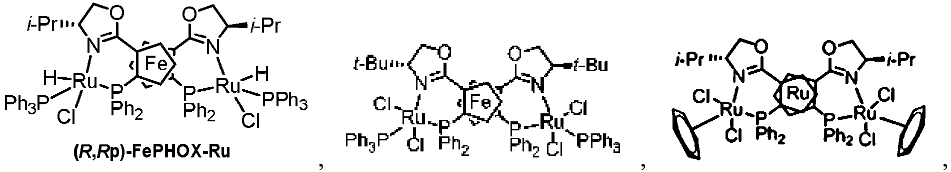

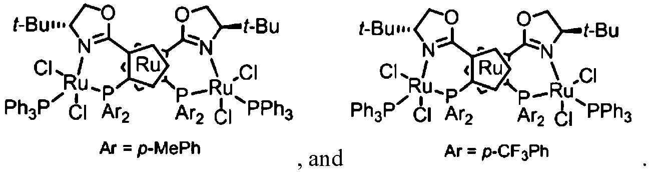

It should be understood that within the scope of the present invention, the above-described technical features of the present invention and the technical features described in detail below (e.g., examples) may be combined with each other to constitute a new or preferred technical solution. Limited by space, it will not be repeated here.

### DESCRIPTION OF THE DRAWINGS

The following drawings are used to illustrate specific embodiments of the present invention and are not intended to limit the scope of the invention as defined by the claims.

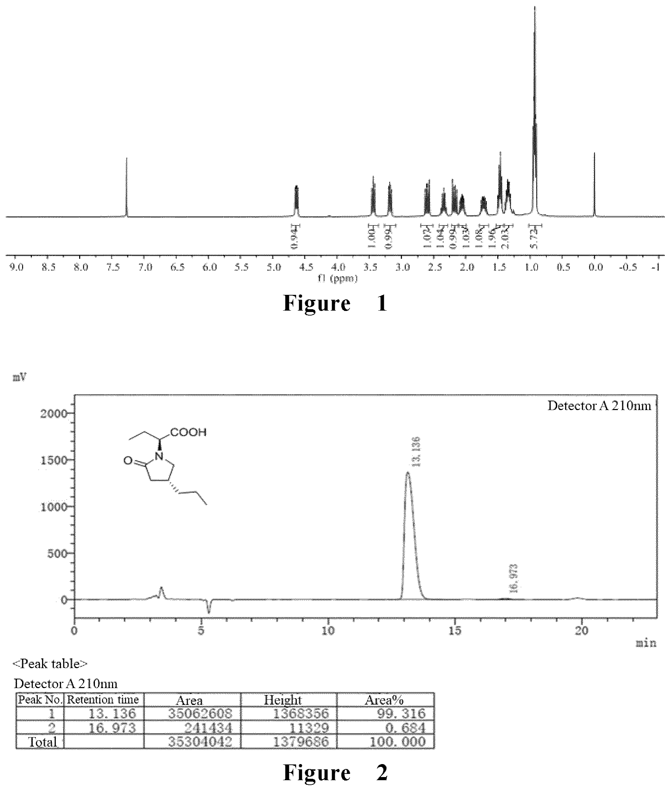

Figure 1 is the HNMR spectrum of (2S)-2-[(4R)-2-oxo-4-propyl-1-pyrrolidinyl] butyric acid obtained in Example 1.
Figure 2 is the HPLC chromatogram of (2S)-2-[(4R)-2-oxo-4-propyl-1-pyrrolidinyl] butyric acid obtained in Example 1. Wherein, 99:1 dr; HPLC [DAICEL CHIRALPAK IK-H, n-hexane/EtOH/MeOH/HCOOH = 94/5/1/0.1,210 nm, 1.0 mL/min, 30°C, t_{R1} = 13.1 min (major), t_{R2} =16.9 min (minor)].
Figure 3 is the HPLC chromatogram of (2S)-2-[(4R)-2-oxo-4-propyl-1-pyrrolidinyl] butanamide obtained in Example 2.

### DETAILED DESCRIPTION OF THE INVENTION

For the deficiencies in the preparation process of brivaracetam and the intermediates thereof in prior arts, the inventors of the present invention have conducted extensive and in-depth research, and developed a new asymmetric catalytic hydrogenation synthesis method for (2S)-2-[(4R)-2-oxo-4-propyl-1-pyrrolidinyl] butyric acid (compound shown in formula II). This synthesis method uses a catalyst formed by a planar chiral metallocene ligand and a ruthenium salt to catalyze the asymmetric hydrogenation of 2S-2-(4-propyl-1,5-dihydropyrrol-2-one) butyric acid (compound shown in formula I), resulting in the final product with a high conversion rate and a high dr value.

### Preparation of catalyst

The method for generating a catalyst by reacting a planar chiral ruthenocene ligand with a ruthenium salt comprises reacting a planar chiral ruthenocene ligand with a ruthenium salt in a solvent at 60-120 °C for 4-8 hours, treating and separating the reaction solution to obtain the catalyst, and more preferably the reaction temperature is 70-100 °C, and the reaction time is 5-7 hours. The solvent used includes but is not limited to methanol, ethanol, isopropanol, propanol, butanol, isobutanol, acetone, 1,4-dioxane, tetrahydrofuran, dichloromethane, acetonitrile, toluene, xylene.

The method for generating a catalyst by reacting a planar chiral ferrocene ligand with a ruthenium salt comprises reacting a planar chiral ferrocene ligand with a ruthenium salt in a solvent at 60-120 °C for 0.3-1 hour to generate a catalyst and obtain a reaction solution containing the catalyst. More preferably the reaction temperature is 70-100 °C and the reaction time is 0.4-0.6 hours. The solvent used includes but is not limited to methanol, ethanol, isopropanol, propanol, butanol, isobutanol, acetone, 1,4-dioxane, tetrahydrofuran, dichloromethane, acetonitrile, toluene, xylene.

When a catalyst is formed by a planar chiral metallocene ligand molecule and two ruthenium salts, in the reaction system where the planar chiral metallocene ligand react with the ruthenium salt to generate a catalyst, the molar ratio of the planar chiral metallocene ligand to the ruthenium salt is 1:2-3, and more preferably 1:2-2.5.

When a catalyst is formed by a planar chiral metallocene ligand molecule and one ruthenium salt, in the reaction system where the planar chiral metallocene ligand react with the ruthenium salt to generate a catalyst, the molar ratio of the planar chiral metallocene ligand to the ruthenium salt is 1:1-2, and more preferably 1:1-1.5.

### Asymmetric catalytic hydrogenation synthesis method of compound shown in formula II

The asymmetric catalytic hydrogenation synthesis method for the compound shown in formula II of the present invention comprises: in a hydrogen atmosphere, in a solvent, the compound represented by formula I is subjected to asymmetric hydrogenation reduction in the presence of an alkali and a catalyst which is formed by a planar chiral metallocene ligand and a ruthenium salt to form a compound as represented by formula II, wherein the reaction formula is shown below: 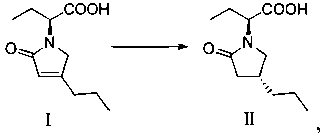 the planar chiral metallocene ligand is selected from compound represented by the following formulas III to IV: 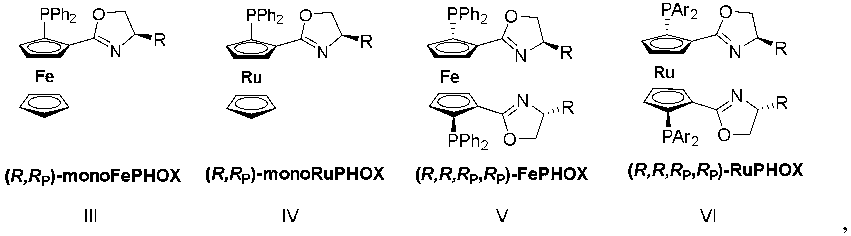 in formulas III-VI, R is selected from H, linear or branched alkyl containing 1-8 carbon atoms, and substituted or unsubstituted aryl or benzyl containing 6-10 carbon atoms; in formula VI, Ar is a substituted or unsubstituted aryl containing 6-10 carbon atoms. The ruthenium salt includes but is not limited to tris (triphenylphosphine) ruthenium chloride, dichloro (p-methylisopropylphenyl) ruthenium (II) dimer, phenylruthenium (II) chloride dimer, bis(2,2'-bipyridyl) ruthenium dichloride, dichloro(1,5-cyclooctadiene)ruthenium (II), benzylidene-bis(tricyclohexylphosphine)dichlororuthenium, p-isopropylphenyl triphenylphosphine ruthenium chloride, bis (triphenylphosphine) cyclopentadienyl ruthenium chloride, chloro (indenyl) bis (triphenylphosphine) ruthenium, pentamethyl cyclopentadienyl bis (triphenylphosphine) ruthenium chloride, dichlorodicarbonyl bis (triphenylphosphine) ruthenium, dichlorobis [(2-propylphosphine) ethylamine] ruthenium, carbonyl chlorohydrotris (triphenylphosphine) ruthenium. More preferably, the catalyst is the compound generated by the compound represented by formula IV or VI and the above ruthenium salt. More preferably, the catalyst is the compound generated by the compound represented by formula VI and tris (triphenylphosphine) ruthenium chloride, dichloro (p-methylisopropylphenyl) ruthenium dimer, or phenylruthenium chloride dimer. Most preferably, the catalyst used in the present invention has a structure as follows:

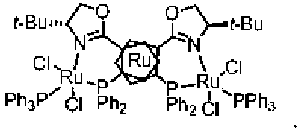

The solvent used in the synthesis reaction includes but is not limited to methanol, ethanol, isopropanol, propanol, butanol, isobutanol, acetone, 1,4-dioxane, tetrahydrofuran, dichloromethane, acetonitrile, toluene, xylene, and more preferably protic solvent such as methanol, ethanol, isopropanol. The alkali used in the synthesis reaction includes but is not limited to LiOH, NaOH, KOH, Li₂CO₃, Na₂CO₃, K₂CO₃, Cs₂CO₃, LiHCO₃, NaHCO₃, KHCO₃, CsHCO₃, Na₃PO₄, Na₂HPO₄, NaH₂PO₄, triethylamine, pyridine, 2,6-methylpyridine, 1,8-diazabicyclo[5,4,0]-7-undecene, and 1,4-diaza[2.2.2]bicyclooctane, and more preferably, inexpensive inorganic base such as, NaOH, KOH. The amount of the alkali used is preferably 0.1-3:1 in molar ratio to the catalytic substrate, and more preferably 0.8-1.5:1. The preferred reaction temperature for asymmetric hydrogenation is -20-60 °C, and more preferably 15-50 °C; and the hydrogen pressure during the reaction process is preferably 1-80 bar, and more preferably 40-60 bar. The amount of catalyst used is not particularly limited, as long as it can significantly promote asymmetric catalytic hydrogenation reaction. From the perspective of efficiency and cost in actual experiments, the molar ratio of the reaction substrate to the catalyst can be 10000:1-10:1, preferably 3000:1-100:1, and more preferably 2000:1-100:1, such as 2000:1, 1500:1, 1000:1, 500:1, and 100:1.

In the asymmetric catalytic hydrogenation synthesis method of (2S)-2-[(4R)-2-oxo-4-propyl-1-pyrrolidinyl] butyric acid of the present invention, the catalyst used can be pre-generated by reacting a planar chiral metallocene ligand with a ruthenium salt before the hydrogenation reaction, and then separated and purified for asymmetric hydrogenation catalysis of the compound shown in formula I. Alternatively, the compound shown in formula I can be added to a reaction system containing a catalyst generated by the reaction of a chiral metallocene ligand and a ruthenium salt, and the asymmetric hydrogenation catalysis reaction can be carried out in situ (the obtained catalyst is not separated and purified). That is, preparation of the catalyst by reacting the planar chiral metallocene ligand with the ruthenium salt and the asymmetric hydrogenation of the substrate can be carried out by a "one-pot process". Here, the "one-pot process" refers to a two-step reaction in which the product obtained in the first step does not require purification, and the raw materials, other additives, and solvent of the second step are directly added to the reaction solution obtained in the first step reaction.

In the description of the present invention, the term "substituted" refers to one or more hydrogen atoms on a functional group being optionally substituted by alkyl, haloalkyl (e.g. trifluoromethyl), alkoxy, cycloalkyl, aryl, benzyl, or halogen (e.g. fluorine, chlorine, bromine, iodine). More preferably, "substituted" means that one or more hydrogen atoms on the aryl or benzyl can be optionally substituted by alkyl containing 1-8 carbon atoms, halogenated alkyl containing 1-8 carbon atoms, alkoxy containing 1-8 carbon atoms, cycloalkyl containing 1-8 carbon atoms, aryl containing 6-10 carbon atoms, benzyl, or halogen.

The term "aryl" refers to a monocyclic or bicyclic hydrocarbon ring system containing at least one unsaturated aromatic ring. For example, phenyl, naphthyl, indolyl, indenyl, etc.

Compared to prior art, the advantageous effects of the present invention are:
1. The catalyst used in the asymmetric catalytic hydrogenation synthesis method of the present invention has good catalytic effect, especially when using the catalyst formed by a planar chiral ruthenocene ligand and a ruthenium salt, the substrate conversion rate is high, the stereoselectivity is good, and the dr value is high;
2. The cost of the catalyst used is lower than that of commonly used palladium catalysts in prior art.

The present invention will be further elaborated below in combination with specific examples. It should be understood that these examples are only used to illustrate the present invention and not to limit the scope of the present invention. In the following examples, the test methods without specific conditions are usually in accordance with conventional conditions or the conditions recommended by the manufacturer. Unless otherwise specified, percentages and parts are calculated by weight.

The reagents and raw materials used in the present invention are commercially available unless otherwise specified.

The catalysts used in Examples 1 to 34, as well as the planar chiral metallocene ligands and ruthenium salts used to form the catalyst are shown in Table 1.

**Table 1**

| Number | Ligand | Ruthenium salt | Catalyst |
|---|---|---|---|
| 1 | 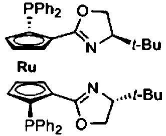 **(*R,R,R*_{P},*R*_{P})-RuPHOX** | Tris (triphenylphosphine) ruthenium chloride | 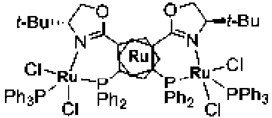 ***(*R*,R,R*_{P},*R*_{P})-RuPHOX-Ru** |
| | Ligand VI-1 | | Catalyst 1 |
| 2 | 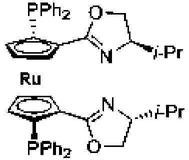 **(*R*,*R*,*R*_{P},*R*ₚ)-RuPHOX** | Tris (triphenylphosphine) ruthenium chloride | 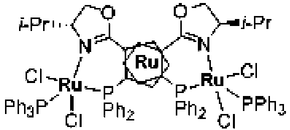 **(*R,R,R*_{P},*R*_{P})-RuPHOX-Ru** |
| | Ligand VI-2 | | Catalyst 2 |
| 3 | 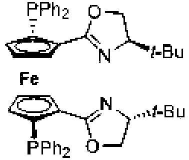 **(*R*,*R,R*_{P},*R*_{P})-FePHOX** | Tris (triphenylphosphine) ruthenium chloride | 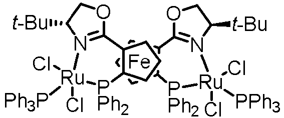 **(*R,R,R*_{P},*R*_{P})-FePHOX-Ru** |
| | Ligand V-1 | | Catalyst 3 |
| 4 | 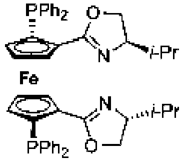 **(*R*,*R*,*R*_{P},*R*_{P})-FePHOX** | Tris (triphenylphosphine) ruthenium chloride | 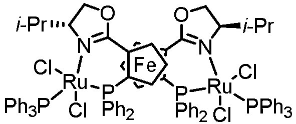 **(*R*,*R*,*R*p,*R*p)-FePHoX-Ru** |
| | Ligand V-2 | | Catalyst 4 |
| 5 | 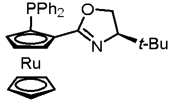 Ligand IV-1 | Tris (triphenylphosphine) ruthenium chloride | 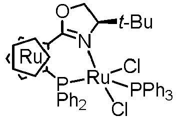 **(R,Rp)-RuPHOX-Ru** |
| | | | Catalyst 5 |
| 6 | 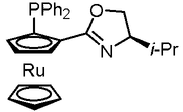 | Tris (triphenylphosphine) ruthenium chloride | 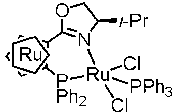 **(R,Rp)-RuPHOX-Ru** |
| | Ligand IV-2 | | Catalyst 6 |
| 7 | 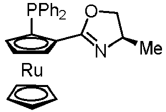 Ligand IV-3 | Tris (triphenylphosphine) ruthenium chloride | 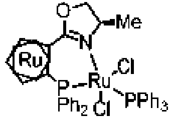 Catalyst 7 |
| 8 | 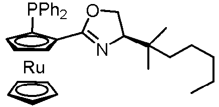 Ligand IV-4 | Tris (triphenylphosphine) ruthenium chloride | 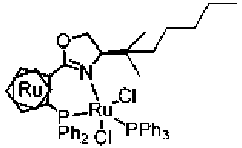 Catalyst 8 |
| 9 | 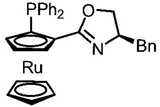 Ligand IV-5 | Tris (triphenylphosphine) ruthenium chloride | 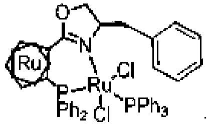 Catalyst 9 |
| 10 | 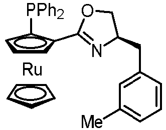 Ligand IV-6 | Tris (triphenylphosphine) ruthenium chloride | 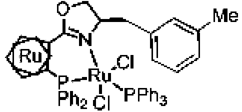 Catalyst 10 |
| 11 | 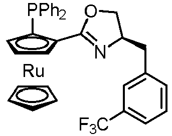 Ligand IV-7 | Tris (triphenylphosphine) ruthenium chloride | 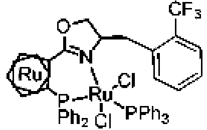 Catalyst 11 |
| 12 | 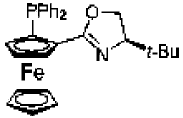 Ligand III-1 | Tris (triphenylphosphine) ruthenium chloride | 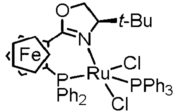 **(*R*,*R*p)-FePHOX-Ru** |
| | | | Catalyst 12 |
| 13 | 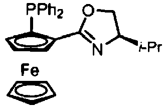 Ligand III-2 | Tris (triphenylphosphine) ruthenium chloride | 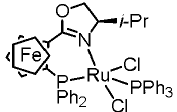 **(*R*,*R*p)-FePHOX-Ru** |
| | | | Catalyst 13 |
| 14 | 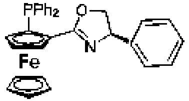 Ligand III-3 | Tris (triphenylphosphine) ruthenium chloride | 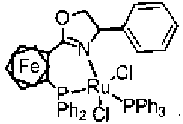 Catalyst 14 |
| 15 | 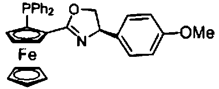 Ligand III-4 | Tris (triphenylphosphine) ruthenium chloride | 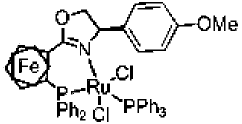 Catalyst 15 |
| 16 | 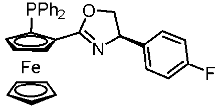 Ligand III-5 | Tris (triphenylphosphine) ruthenium chloride | 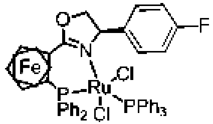 Catalyst 16 |
| 17 | 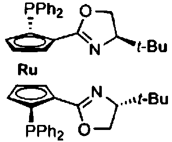 **(*R*,*R*,*R*_{P},*R*_{P})-RuPHOX** | Dichloro (p-methylisopropylp henyl) ruthenium (II) dimer | 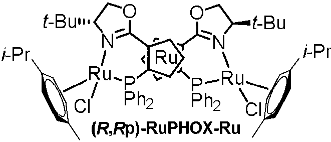 |
| | Ligand VI-1 | | Catalyst 17 |
| 18 | 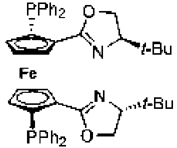 **(*R*,*R*,*R*_{P},*R*_{P})-FePHOX** | Dichloro (p-methylisopropylp henyl) ruthenium (II) dimer | 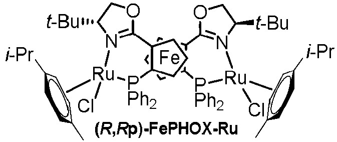 Catalyst 18 |
| | Ligand V-1 | | |
| 19 | 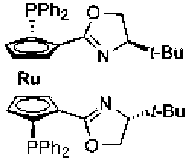 **(*R*,*R,R*_{P},*R*_{P})-RuPHOX** | Phenylruthenium (II) chloride dimer | 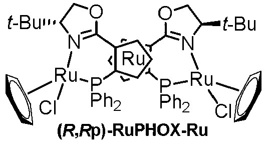 Catalyst 19 |
| | Ligand VI-1 | | |
| 20 | 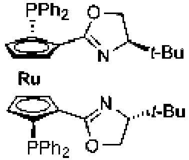 **(*R*,*R*,*R*_{P},*R*_{P})-RuPHOX** | Bis(2,2'-bipyridyl) ruthenium dichloride | 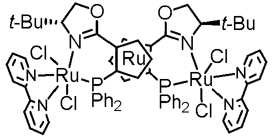 **(*R*,*R*p)-RuPHOX-Ru** |
| | Ligand VI-1 | | Catalyst 20 |
| 21 | 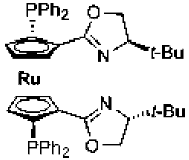 **(*R,R,R*_{P},R_{P})-RuPHOX** | Dichloro(1,5-cyclooc tadiene)ruthenium | 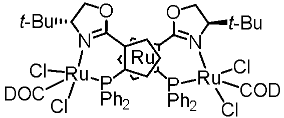 **(*R*,*R*p)-RuPHOX-Ru** |
| | Ligand VI-1 | | Catalyst 21 |
| 22 | 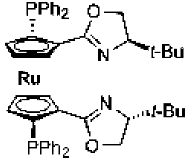 **(*R,R,R*_{P},*R*_{P})-RuPHOX** | Tris (tri p-phenylmethylphos phine) ruthenium chloride | 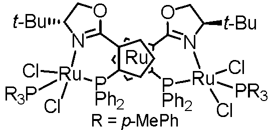 **(*R*,*R*p)-RuPHOX-Ru** |
| | Ligand VI-1 | | Catalyst 22 |
| 23 | 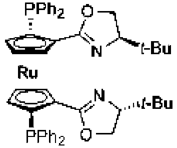 **(*R,R,R*_{P},*R*_{P})-RuPHOX** | Tris (tri 4-trifluoromethylphe nylphosphine) ruthenium chloride | 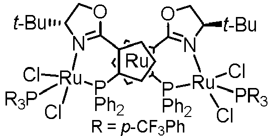 **(*R*,*R*p)-RuPHOX-Ru** |
| | Ligand VI-1 | | Catalyst 23 |
| 24 | 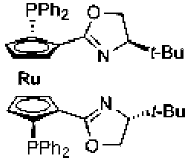 **(*R*,*R*,*R*ₚ,*R*ₚ)-RuPHOX** | Bis (triphenylphosphine) cyclopentadienyl ruthenium chloride (II) | 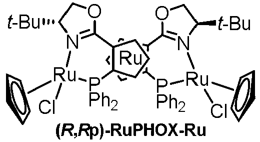 Catalyst 24 |
| | Ligand VI-1 | | |
| 25 | 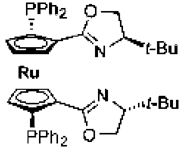 **(*R,R,R*_{P},*R*_{P})-RuPHOX** | Chloro (indenyl) bis (triphenylphosphine) ruthenium (II) | 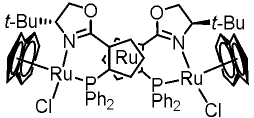 **(*R*,*R*p)-RuPHOX-Ru** 25 |
| | Ligand VI-1 | | Catalyst |
| 26 | 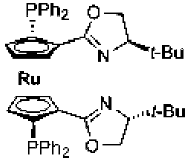 **(*R*,*R*,*R*_{P},*R*_{P})-RuPHOX** | Pentamethyl cyclopentadienyl bis (triphenylphosphine) ruthenium chloride (II) | 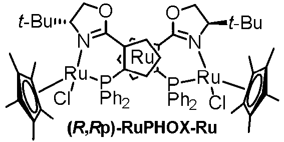 Catalyst 26 |
| | Ligand VI-1 | | |
| 27 | 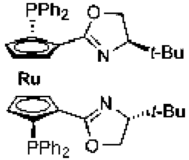 **(*R,R,R*_{P},*R*_{P})-RuPHOX** | Dichlorodicarbonyl bis (triphenylphosphine) ruthenium | 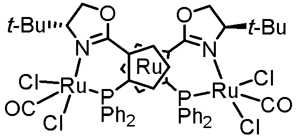 **(*R*,*R*p)-RuPHOX-Ru** |
| | Ligand VI-1 | | Catalyst 27 |
| 28 | 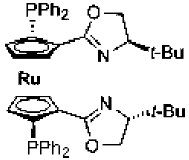 **(*R,R,R*_{P},*R*_{P})-RuPHOX** | Dichlorobis [(2-propylphosphine) ethylamine] ruthenium (II) | 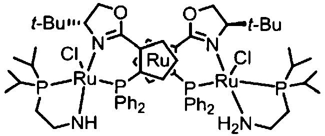 **(*R*,*R*p)-RuPHOX-Ru** |
| | Ligand VI-1 | | Catalyst 28 |
| 29 | 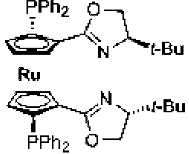 **(*R,R,R*_{P},*R*_{P})-RuPHOX** | Carbonyl chlorohydrotris (triphenylphosphine) ruthenium (II) | 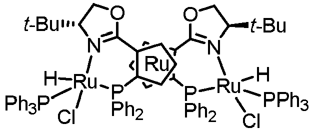 **(*R*,*R*p)-RuPHOX-Ru** |
| | Ligand VI-1 | | Catalyst 29 |
| 30 | 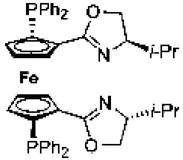 **(*R,R,R*_{P},*R*_{P})-FePHOX** | Carbonyl chlorohydrotris (triphenylphosphine) ruthenium (II) | 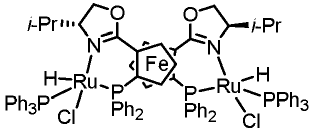 **(*R*,*R*p)-FePHOX-Ru** |
| | Ligand V-2 | | Catalyst 30 |
| 31 | 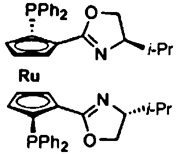 **(*R*,*R*,*R*_{P},*R*_{P})-RuPHOX** | Dichloro (p-methylisopropylp henyl) ruthenium (II) dimer | 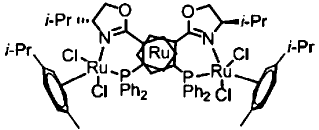 **(*R*,*R*p)-RuPHOX-Ru** |
| | Ligand VI-2 | | Catalyst 31 |
| 32 | 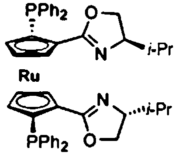 **(*R*,*R*,*R*ₑ,*R*ₚ)-RuPHOX** | Phenylruthenium (II) chloride dimer | 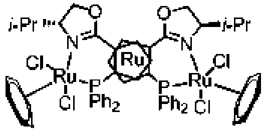 **(*R,R*p)-RuPHOX-Ru** |
| | Ligand VI-2 | | Catalyst 32 |
| 33 | 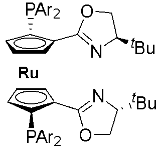 Ar = p-MePh | Tris (triphenylphosphine) ruthenium chloride | 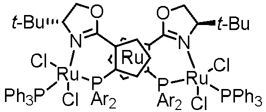 Ar = p-MePh |
| | **(*R,R,R*_{P},*R*_{P})-RuPHOX** | | **(*R,R,R*_{P},*R*_{P})-RuPHOX** |
| | Ligand VI-3 | | Catalyst 33 |
| 34 | 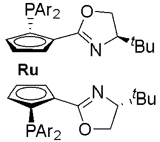 Ar = *p*-CF₃Ph | Tris (triphenylphosphine) ruthenium chloride | 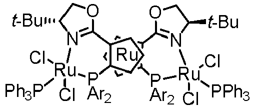 Ar = *p*-CF₃Ph |
| | **(*R,R,R*_{P},*R*_{P})-RuPHOX** | | **(*R,R,R*_{P},*R*_{P})-RuPHOX** |
| | Ligand VI-4 | | Catalyst 34 |

The raw materials used in the following examples, such as the compound shown in formula I, have an ee value greater than 99%.

### Example 1:

### 1.1 Typical operations for catalyst preparation

Ligand VI-1 (69.5 mg) and tris (triphenylphosphine) ruthenium chloride (156.8 mg) were dissolved in toluene (25 mL) and the mixture was heated and stirred at 90 °C for 6 hours. The reaction solution was cooled to room temperature, the solvent was removed under reduced pressure, and the resulting residue was separated by column chromatography [eluent: V (petroleum ether): V (ethyl acetate) = 5:1] to obtain a dark green solid (136.0 mg, 96.8%), i.e., catalyst 1.

The HNMR data of catalyst 1 were as follows:
¹H NMR (500 MHz, CDCl₃):7.66-6.75 (m, 50H), 5.60-5.50 (m, 2H), 5.43 (s, 2H),4.93 (t, *J*=10.0 Hz, 2H), 4.70-4.63 (m, 2H), 4.45 (s,2H), 4.40 (t, *J*=9.0 Hz, 2H), 0.79 (s, 18H); ³¹PNMR (162 MHz, CDCl₃) *δ*: 67.53, 42.63.

### 1.2 Preparation of (2S)-2-[(4R)-2-oxo-4-propyl-1-pyrrolidinyl] butyric acid

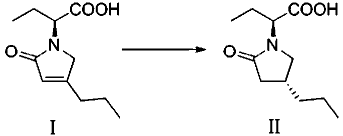

Compound shown in formula I (1.0g, 4.7 mmol), NaOH (0.2g, 5.2 mmol, 1.1 equiv), and catalyst 1 (4.1 mg, 2.35 µmol, 0.05 mol%) were added sequentially into a 50 mL dry round-bottom flask equipped with a magnetic stirrer. After the atmosphere was vacuumed and replaced by nitrogen, 10 mL of degassed isopropanol was added, the mixture was stirred well (the solution turned green), and the round-bottom flask was placed in the hydrogenation reactor. After the atmosphere in the hydrogenation reactor was replaced by hydrogen three times, hydrogen was added to a pressure of 50 bar. After stirred in an oil bath at 35 °C for 48 hours, the reaction mixture was cooled to room temperature, hydrogen gas was slowly released, and the solvent was evaporated under reduced pressure. The reaction mixture was diluted with water (10 mL) and adjusted to pH=1 with dilute hydrochloric acid, and extracted with ethyl acetate (20mL × 3). The organic phase was dried over anhydrous sodium sulfate, and filtered, and the solvent was evaporated to obtain crude (2S)-2-[(4R)-2-oxo-4-propyl-1-pyrrolidinyl] butyric acid (>1.0g, dr value greater than 99:1).

The crude product was purified by recrystallization using methyl *tert*-butyl ether: cyclohexane (volume ratio of 1:5) to obtain the pure product. The HNMR of pure product was as follows:
¹H NMR (400 MHz, CDCl₃)*δ*: 4.63 (dd, *J* = 10.8, 5.2 Hz, 1H), 3.44 (dd, *J* = 9.4, 7.8 Hz, 1H), 3.18 (dd, *J* = 9.4, 7.2 Hz, 1H), 2.60 (dd, *J* = 16.8, 8.4 Hz, 1H), 2.42-2.28 (m, 1H), 2.17 (dd, *J =* 16.8, 8.4 Hz, 1H), 2.11-1.99 (m, 1H), 1.79-1.65 (m, 1H), 1.51-1.43 (m, 2H), 1.39-1.29 (m, 2H), 0.98-0.88 (m, 6H).

The crude product was tested by HPLC, and the HPLC information was as follows:
[DAICEL CHIRALPAK IK-Hcolumn, n-hexane/EtOH/MeOH/HCOOH = 94/5/1/0.1, 210nm, 1.0 mL/min, 30 °C, t_{R1} = 13.1 min (major), t_{R2} =16.9 min (minor)], dr > 99:1.

### Example 2:

### Preparation of (2S)-2-[(4R)-2-oxo-4-propyl-1-pyrrolidinyl] butanamide

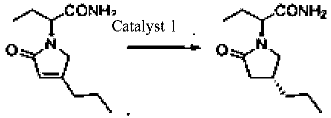

2S-2-(4-propyl-1,5-dihydropyrrol-2-one) butanamide (1.0g, 4.7 mmol, ee greater than 99%), Cs₂CO₃ (3.1g, 9.4 mmol, 2.0 equiv), and catalyst 1 (4.1 mg, 2.35 µmol, 0.05mol%) were added sequentially into a 50 mL dry round-bottom flask equipped with a magnetic stirrer. After the atmosphere was vacuumed and replaced by nitrogen, 10 mL of degassed isopropanol was added, the mixture was stirred well (the solution turned green), and the round-bottom flask was placed in the hydrogenation reactor. After the atmosphere in the hydrogenation reactor was replaced by hydrogen three times, hydrogen was added to a pressure of 40 bar. After stirred at room temperature for 48 hours, the reaction mixture was cooled to room temperature, and hydrogen gas was slowly released. The solvent was evaporated under reduced pressure, the reaction mixture was diluted with water (10 mL) and adjusted to pH=1 with dilute hydrochloric acid, and extracted with ethyl acetate (20mL × 3). The organic phase was dried over anhydrous sodium sulfate, and filtered, and the solvent was evaporated to obtain the residue (substrate conversion rate>99% conv. ,> 1.0 g, dr value is 1.2:1).

The above residue was separated by a chiral column to obtain (2S)-2-[(4R)-2-oxo-4-propyl-1-pyrrolidinyl] butanamide. The HNMR was as follows:
¹H NMR (400 MHz, CDCl₃) δ: 6.44 (s, 1H), 5.78 (s, 1H), 4.45-4.42 (m, 1H), 3.49 (dd, *J =* 9.6, 7.6 Hz, 1H), 3.05 (dd, *J =* 9.6, 6.8 Hz, 1H), 2.55 (dd, *J =* 16.8, 8.4 Hz, 1H),2.40-2.28 (m, 1H), 2.12-2.07 (m,1H), 1.99-1.88 (m, 1H), 1.73-1.62 (m, 1H), 1.46-1.38 (m, 2H), 1.37-1.29 (m, 2H), 0.95-0.86 (m, 6H).

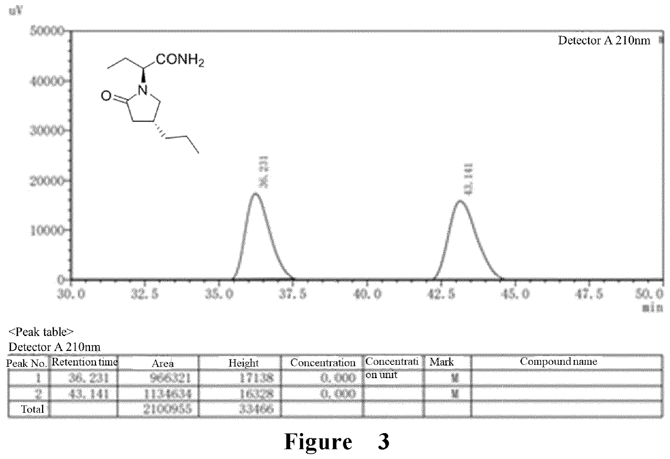

The crude product was detected by HPLC, and the result was shown in Figure 3.

### Example 3

### 3.1 Preparation of catalyst

Ru(II)Cl₂(PPh₃)₃ (4.5 mg, 0.1 mol%) and ligand V-1 (2.3 mg, 0.06 mol%) were added to a 50 mL two-necked flask, and isopropanol (5 mL) was added under nitrogen atmosphere. The reaction system was frozen by liquid nitrogen, vacuumed, and the atmosphere was replaced by nitrogen three times. Then, the mixture was refluxed for 0.5 h. During this process, the brown insoluble substance gradually was dissolved to form a homogeneous solution, resulting in catalyst 3.

### 3.2 Preparation of compound shown in formula II

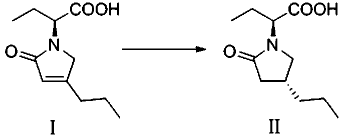

Compound shown in formula I (1.0g, 4.7 mmol), NaOH (0.2g, 5.2 mmol, 1.1 equiv), and degassed isopropanol (10 mL) were added to the reaction system containing catalyst 3, the mixture was stirred well, and the round-bottom flask was placed in the hydrogenation reactor. After the atmosphere in the reactor was replaced by hydrogen three times, hydrogen was added to a pressure of 50 bar. After stirred in an oil bath at 50 °C for 48 hours, the reaction mixture was cooled to room temperature, hydrogen gas was slowly released, and the solvent was evaporated under reduced pressure. The residue was diluted with water (10 mL) and adjusted to pH=1 with dilute hydrochloric acid, and extracted with ethyl acetate (20mL × 3). The organic phase was dried over anhydrous sodium sulfate, and filtered, and the solvent was evaporated to obtain crude compound shown in formula II (substrate conversion rate >1.0 g, dr value was 7:1).

The crude product was tested by HPLC, and the HPLC information was as follows:
[DAICEL CHIRALPAK IK-Hcolumn, n-hexane/EtOH/MeOH/HCOOH = 94/5/1/0.1, 210nm, 1.0 mL/min, 30 °C, t_{R1}=13.1 min (major), t_{R2}=16.9 min (minor)], dr was approximately 7:1.

### Examples 4-33

The preparation method of the catalysts formed by the planar chiral ruthenocene ligands and ruthenium salts used in the following Examples 4 to 33 was similar to that in Example 1 (the molar ratio of ligand to ruthenium salt was adjusted according to the ratio of the two molecules in the catalyst structure), and these catalysts catalyzed the hydrogenation of the compound shown in Formula I in a similar manner to Example 1. The preparation method of the catalyst formed by the planar chiral ferrocene ligand and ruthenium salt used is similar to that in Example 3, (the molar ratio of ligand to ruthenium salt was adjusted according to the ratio of the two molecules in the catalyst structure), and these catalysts catalyzed the hydrogenation of the compound shown in Formula I in a similar manner to Example 3.

### Typical operation for preparation of catalyst 5

Ligand IV-1 (540 mg) and tris (triphenylphosphine) ruthenium chloride (1054 mg) were dissolved in toluene (25 mL) and the mixture was heated and stirred at 90 °C for 6 hours. The reaction solution was cooled to room temperature, the solvent was removed under reduced pressure, and the resulting residue was separated by column chromatography [eluent: V (petroleum ether): V (ethyl acetate)=5:1] to obtain a dark green solid (935 mg, 95% yield), i.e., catalyst 5.

Catalyst 5 catalyzed the hydrogenation of the compound shown in formula I in a similar manner to Example 1.

### Typical operation for the preparation of catalyst 12

Ru(II)Cl₂(PPh₃)₃ (4.5 mg, 0.1 mol%) and ligand III-1 (2.3 mg, 0.1 mol%) were added to a 50 mL two-necked flask, and isopropanol (2 mL) was added under nitrogen atmosphere. The reaction system was frozen by liquid nitrogen, vacuumed, and the atmosphere was replaced by nitrogen three times. Then, the mixture was refluxed for 0.5 h. During this process, the brown insoluble substance gradually was dissolved to form a homogeneous solution, obtaining a solution containing catalyst 12.

Catalyst 12 catalyzed the hydrogenation of the compound shown in formula I in a similar manner to Example 3.

The catalysts used in the following Examples 4-33 and reaction results were shown in Table 2, wherein the molar ratio of the substrate (compound shown in Formula I) to the catalyst was approximately 2000:1 (the dr values in Table 2 were all for crude products).

**Table 2**

| Example | Catalyst | Solvent | Tempe rature | Alkali | Hydrogen Pressure (bar) | Conversion Rate (%) | dr |
|---|---|---|---|---|---|---|---|
| 4 | Catalyst 2 | MeOH | 30 | Na₂CO₃ | 50 | >99 | >99:1 |
| 5 | Catalyst 4 | EtOH | 30 | Na₂CO₃ | 50 | >99 | 81:19 |
| 6 | Catalyst 5 | EtOH | 25 | KOH | 40 | 96 | 85:15 |
| 7 | Catalyst 6 | EtOH | 25 | NaOH | 40 | 95 | 86:14 |
| 8 | Catalyst 7 | *n*-BuOH | 25 | NaOH | 40 | 96 | 92:8 |
| 9 | Catalyst 8 | EtOH | 35 | KOH | 40 | 97 | 89:11 |
| 10 | Catalyst 9 | EtOH | 25 | NaOH | 40 | 98 | 90:10 |
| 11 | Catalyst 10 | EtOH | 25 | KOH | 40 | 97 | 87:13 |
| 12 | Catalyst 11 | *i*-PrOH | 25 | Na₂CO₃ | 50 | 95 | 85:15 |
| 13 | Catalyst 12 | MeOH | 30 | Na₂CO₃ | 50 | 93 | 77:23 |
| 14 | Catalyst 13 | MeOH | 30 | Na₂CO₃ | 50 | 98 | 80:20 |
| 15 | Catalyst 14 | MeOH | 30 | Na₂CO₃ | 50 | 97 | 83:17 |
| 16 | Catalyst 15 | MeOH | 25 | Na₂CO₃ | 50 | 94 | 80:20 |
| 17 | Catalyst 16 | *i*-PrOH | 25 | NaOH | 50 | 92 | 81:11 |
| 18 | Catalyst 17 | MeOH | 25 | Na₂CO₃ | 60 | 95 | >99:1 |
| 19 | Catalyst 18 | *i*-PrOH | 30 | NaOH | 50 | 96 | 79:21 |
| 20 | Catalyst 19 | *n*-PrOH | 35 | LiOH | 50 | 96 | 91:9 |
| 21 | Catalyst 20 | *i*-PrOH | 50 | KOH | 50 | 91 | 88:12 |
| 22 | Catalyst 21 | *i*-PrOH | 40 | Na₂CO₃ | 60 | 99 | >99:1 |
| 23 | Catalyst 22 | *i*-BuOH | 40 | NaOH | 50 | 98 | 98:2 |
| 24 | Catalyst 23 | *i*-PrOH | 35 | K₂CO₃ | 60 | 95 | 98:2 |
| 25 | Catalyst 24 | *i*-BuOH | 30 | NaOH | 40 | 68 | 88:12 |
| 26 | Catalyst 25 | *i*-PrOH | 30 | Na₂CO₃ | 50 | 77 | 95:5 |
| 27 | Catalyst 26 | *i*-PrOH | 25 | Cs₂CO₃ | 40 | 59 | 90:10 |
| 28 | Catalyst 27 | *n*-BuOH | 25 | K₂CO₃ | 60 | 50 | 72:28 |
| 29 | Catalyst 28 | EtOH | 25 | NaOH | 40 | 78 | 76:24 |
| 30 | Catalyst 29 | *i*-PrOH | 25 | NaOH | 40 | 88 | 97:3 |
| 31 | Catalyst 30 | *i*-PrOH | 30 | NaOH | 40 | 65 | 84:16 |
| 32 | Catalyst 31 | *i*-PrOH | 30 | NaOH | 40 | 69 | 80:20 |
| 33 | Catalyst 32 | *i*-PrOH | 40 | NaOH | 40 | 87 | 87:13 |
| 34 | Catalyst 33 | *i*-PrOH | 35 | Na₂CO₃ | 60 | 98 | 98:2 |
| 35 | Catalyst 34 | *i*-PrOH | 35 | Na₂CO₃ | 60 | 98 | 98:2 |

From the above examples 4 to 35, it can be seen that the catalysts formed by planar chiral ruthenocene ligands and ruthenium salts have better catalytic selectivity than the catalysts formed by chiral ferrocene ligands and ruthenium salts.

### Examples 36-71

Taking catalyst 1 as the catalyst, the compound shown in formula I was catalyzed to undergo asymmetric hydrogenation, and the temperature, time, hydrogen pressure, solvent, etc. of the catalytic reduction were screened.

The following examples 36-71 used catalyst 1 to asymmetrically catalyze the hydrogenation of the compound shown in formula I to synthesize the compound shown in formula II, wherein the molar ratio of the substrate to the catalyst was approximately 2000:1. The solvent, alkali, reaction system temperature (solvent temperature), hydrogen pressure, and reaction time used, and reaction results (substrate conversion rate, dr value) were shown in Table 3 (dr values in Table 3 were all dr values for crude product).

**Table 3**

| Example | Solvent | Alkali | Solvent temperature (°C) | Hydrogen Pressure (bar) | Time (h) | Conversion Rate (%) | dr |
|---|---|---|---|---|---|---|---|
| 36 | MeOH | NaOH | 25 | 40 | 48 | 85 | 90:10 |
| 37 | EtOH | NaOH | 25 | 40 | 48 | >99 | 95:5 |
| 38 | i-PrOH | NaOH | 25 | 40 | 48 | >99 | 96:4 |
| 39 | n-PrOH | NaOH | 25 | 40 | 48 | >99 | 93:7 |
| 40 | n-BuOH | NaOH | 25 | 40 | 48 | 80 | 90:10 |
| 41 | i-BuOH | NaOH | 25 | 40 | 48 | 86 | 90:10 |
| 42 | Acetone | NaOH | 25 | 40 | 48 | 60 | 75:25 |
| 43 | 1,4-Dioxane | NaOH | 25 | 40 | 48 | 63 | 80:20 |
| 44 | PhMe | NaOH | 25 | 40 | 48 | 78 | 90:10 |
| 45 | DCM | NaOH | 25 | 40 | 48 | 66 | 85:15 |
| 46 | THF | NaOH | 25 | 40 | 48 | 65 | 75:25 |
| 47 | TFE | NaOH | 25 | 40 | 48 | 85 | 88:12 |
| 48 | CH₃CN | NaOH | 25 | 40 | 48 | 55 | 92:8 |
| 49 | *i*-PrOH | LiOH | 25 | 40 | 48 | 95 | 96:4 |
| 50 | *i*-PrOH | KOH | 25 | 40 | 48 | 90 | 96:4 |
| 51 | *i*-PrOH | Na₂CO₃ | 25 | 40 | 48 | 60 | 96:4 |
| 52 | *i*-PrOH | NaHCO₃ | 25 | 40 | 48 | 40 | 70:30 |
| 53 | *i*-PrOH | K₂CO₃ | 25 | 40 | 48 | 60 | 82:18 |
| 54 | *i*-PrOH | Cs₂CO₃ | 25 | 40 | 48 | 60 | 90:10 |
| 55 | *i*-PrOH | LiHCO₃ | 25 | 40 | 48 | 50 | 75:35: |
| 56 | *i*-PrOH | Na₃PO₄ | 25 | 40 | 48 | 45 | 85:25 |
| 57 | *i*-PrOH | Na₂HPO₄ | 25 | 40 | 48 | 60 | 84:26 |
| 58 | *i*-PrOH | Triethylamine | 25 | 40 | 48 | 15 | 55:45 |
| 59 | *i*-PrOH | Pyridine | 25 | 40 | 48 | 10 | 70:30 |
| 60 | *i*-PrOH | 2,6-Methylp yridine | 25 | 40 | 48 | 12 | 60:40 |
| 61 | *i*-PrOH | DBU | 25 | 40 | 48 | 20 | 68:32 |
| 62 | *i*-PrOH | DABCO | 25 | 40 | 48 | 16 | 58:42 |
| 63 | *i*-PrOH | NaOH | 30 | 40 | 48 | >99 | 97:3 |
| 64 | *i*-PrOH | NaOH | 35 | 40 | 48 | >99 | 98:2 |
| 65 | *i*-PrOH | NaOH | 40 | 40 | 48 | >99 | 96:4 |
| 66 | *i*-PrOH | NaOH | 50 | 40 | 48 | >99 | 97:3 |
| 67 | *i*-PrOH | NaOH | 35 | 30 | 48 | 92 | 98:2 |
| 68 | *i*-PrOH | NaOH | 35 | 50 | 48 | >99 | >99:1 |
| 69 | *i*-PrOH | NaOH | 35 | 60 | 48 | >99 | 98:2 |
| 70 | *i*-PrOH | NaOH | 35 | 50 | 24 | 72 | >99:1 |
| 71 | *i*-PrOH | NaOH | 35 | 50 | 36 | 95 | >99:1 |

From the above examples 36-71, it can be seen that catalyst 1 has a good catalytic selectivity when alcohol was chose as the reaction solvent, and inorganic alkali as the alkali at a temperature of about 35 °C and a hydrogen pressure of about 50 bar for about 48 hours.

### Examples 72~77

### Preparation of (2S)-2-[(4R)-2-oxo-4-propyl-1-pyrrolidinyl] butyric acid

The preparation of Examples 72 to 77 was according to the description in 1.2 of Example 1, with the only difference being the molar ratio of the substrate (compound shown in Formula I) to the catalyst (catalyst 1). The specific molar ratio of the substrate to the catalyst and the reaction results were shown in Table 4.

**Table 4**

| Example | Molar ratio of substrate to catalyst | Conversion rate (%) | dr |
|---|---|---|---|
| 72 | 1000:1 | >99 | >99:1 |
| 73 | 1:0.01 | >99 | >99:1 |
| 74 | 10:1 | >99 | >99:1 |
| 75 | 2000:1 | >99 | >99:1 |
| 76 | 3000:1 | >96 | >99:1 |
| 77 | 10000:1 | 84 | >99:1 |

From the above examples 60-65, it can be seen that catalyst 1 can achieve a conversion rate of 84% when NaOH was chose as the alkali, isopropanol as the reaction solvent at a temperature of 35 °C and a hydrogen pressure of 50 bar for 48 hours and the molar ratio of the substrate to the catalyst was 10000:1. When the molar ratio was greater than 1000:1, the conversion rate did not significantly increase. In addition, during the actual experimental process, it was found that as long as the reaction time was appropriately extended, the reaction with a molar ratio of the substrate to the catalyst of 10000:1 can also achieve complete conversion and excellent dr value, and this ratio can still be further increased. However, considering the synthesis cost, the molar ratio of the substrate to the catalyst was preferably between 2000-100:1.

### Example 78

### Synthesis of brivaracetam from the compound of formula II

Esterification reaction: 33mL of methanol was added to a reaction flask, the atmosphere was protected with nitrogen, the methanol was cooled down to -10 °C, and 1.8g of thionyl chloride was added, while controlling the temperature to -10-0 °C to complete the dropwise addition. 2.2g of the compound shown in formula II was added, and the mixture was reacted at -5 °C-0 °C for 3 hours. TLC detected that the reaction was completed, the mixture was concentrated to dryness, ethyl acetate and water were added, and sodium carbonate solution was added to adjust the pH to 6-8. The resulting solution was separated, the organic phase was dried over anhydrous magnesium sulfate, and filtered. The filtrate was concentrated to dryness to obtain 2.5g of yellow oil, i.e., methyl (2S)-2-[(4R)-2-oxo-4-propyl-1-pyrrolidinyl] butyrate. The quality yield was 113.63%. The HPLC purity was 98.22%.

Amination reaction: 25mL of concentrated ammonia water and 2.5mL of ethanol were added to the reaction flask, the mixture was cooled down to 0 °C, and 2.5g of the above yellow oil was added until completely dissolved. Ammonia gas was introduced for ammonification at -5-0 °C. After TLC indicated the completion of the reaction ethyl acetate and water were added, and hydrochloric acid was added to adjust the system pH to 6-8, and the organic phase was separated. The organic phase was dried using anhydrous magnesium sulfate, and filtered. The filtrate was concentrated to dryness, and refined with isopropyl ether to obtain 2.2g of brivaracetam. The quality yield was 88%. The HPLC purity was 100% and the optical purity was 99.93%.

All references mentioned in the present invention are cited as references in this application, as if each reference was cited separately. In addition, it should be understood that after reading the above teaching content of the present invention, those skilled in the art can make various changes or modifications to the present invention, and these equivalent forms also fall within the scope of the claims attached to this application.

## Claims

1. An asymmetric catalytic hydrogenation synthesis method for the compound shown in formula II, wherein the synthesis method comprises the step of: in a hydrogen atmosphere, in a solvent, the compound represented by formula I is subjected to asymmetric hydrogenation reduction in the presence of an alkali and a catalyst which is formed by a planar chiral metallocene ligand and a ruthenium salt, to form a compound as represented by formula II, wherein the reaction formula is shown below: 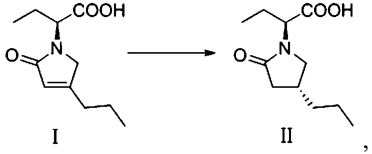
the planar chiral metallocene ligand is selected from the compound represented by the following formulas III to VI: 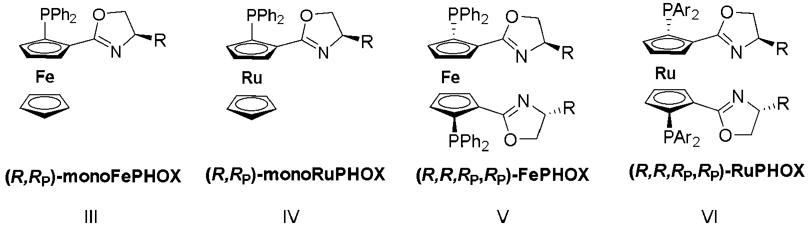
in formulas III-VI, R is selected from H, linear or branched alkyl containing 1-8 carbon atoms, cycloalkyl containing 1-8 carbon atoms, and substituted or unsubstituted aryl or benzyl containing 6-10 carbon atoms, in formula VI, Ar is a substituted or unsubstituted aryl containing 6-10 carbon atoms, and "substituted" means that one or more hydrogen atoms on the aryl or benzyl can be optionally substituted by alkyl, haloalkyl, alkoxy, cycloalkyl, aryl, benzyl or halogen,
the ruthenium salt is selected from the group consisting of tris (triphenylphosphine) ruthenium chloride, dichloro (p-methylisopropylphenyl) ruthenium (II) dimer, phenylruthenium (II) chloride dimer, bis(2,2'-bipyridyl) ruthenium dichloride, dichloro(1,5-cyclooctadiene)ruthenium (II), benzylidene-bis(tricyclohexylphosphine)dichlororuthenium, p-isopropylphenyl triphenylphosphine ruthenium chloride, bis (triphenylphosphine) cyclopentadienyl ruthenium chloride, chloro (indenyl) bis (triphenylphosphine) ruthenium, pentamethyl cyclopentadienyl bis (triphenylphosphine) ruthenium chloride, dichlorodicarbonyl bis (triphenylphosphine) ruthenium, dichlorobis [(2-propylphosphine) ethylamine] ruthenium, carbonyl chlorohydrotris (triphenylphosphine) ruthenium, and the combinations thereof.

2. The asymmetric catalytic hydrogenation synthesis method according to claim 1, wherein in the formulas III-VI, R is selected from the group consisting of methyl, ethyl, isopropyl, tert-butyl, substituted or unsubstituted aryl containing 6-10 carbon atoms, and benzyl, more preferably, isopropyl or tert-butyl, and/or
in formula VI, Ar is phenyl, or phenyl substituted with methyl or trifluoromethyl.

3. The asymmetric catalytic hydrogenation synthesis method according to claim 1, wherein the planar chiral metallocene ligand is selected from compounds represented by Formula IV or VI, wherein, R is selected from the group consisting of methyl, ethyl, isopropyl, *tert-butyl,* aryl containing 6-10 carbon atoms, and benzyl, and/or
the ruthenium salt is selected from the group consisting of tris (triphenylphosphine) ruthenium chloride, dichloro (*p*-methylisopropylphenyl) ruthenium dimer, and phenylruthenium chloride dimer.

4. The asymmetric catalytic hydrogenation synthesis method according to claim 1 or 2, wherein the catalyst is selected from the compounds shown in the following structure: 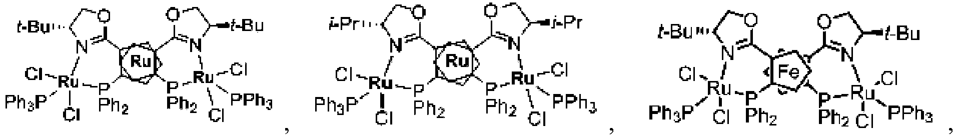 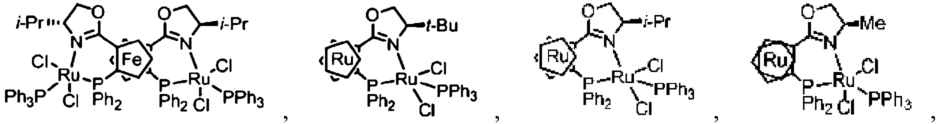 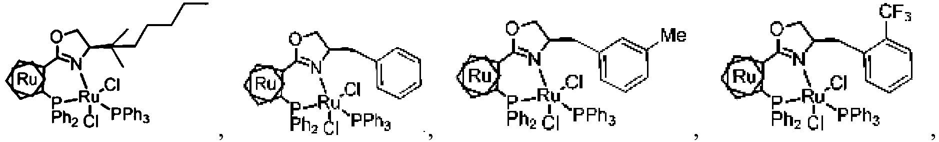 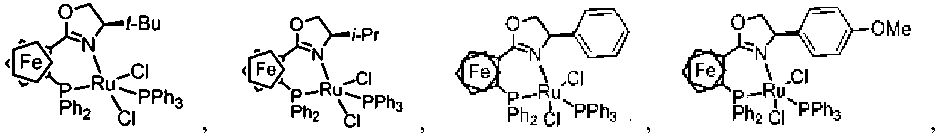 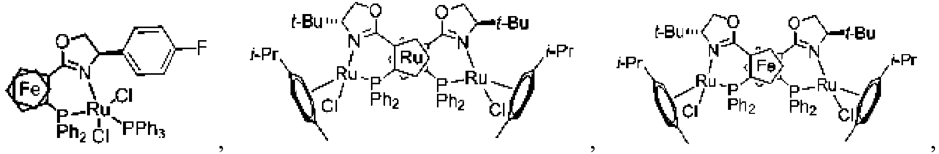 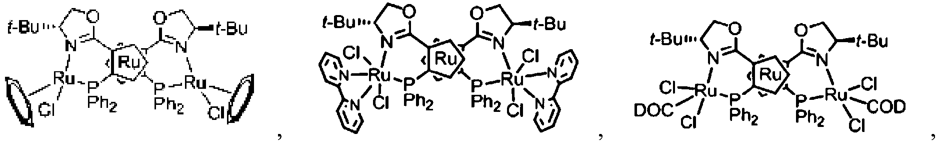 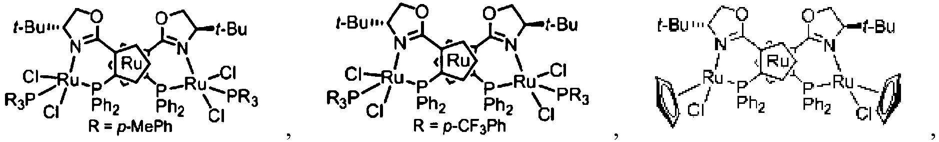 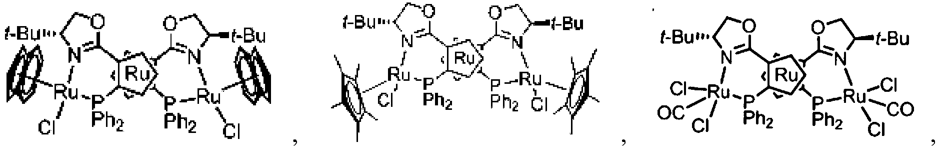 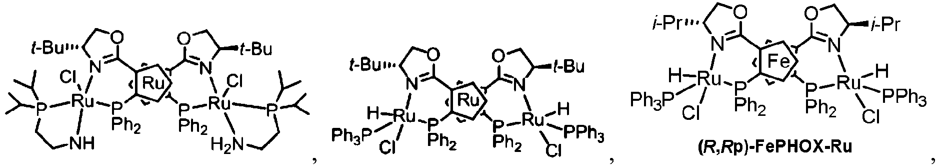 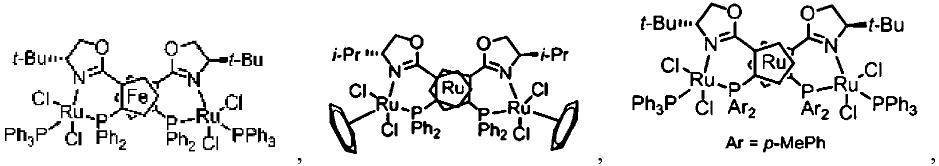 and

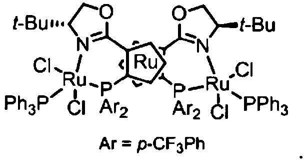

5. The asymmetric catalytic hydrogenation synthesis method according to claim 4, wherein the catalyst is selected from the compounds shown in the following structure: 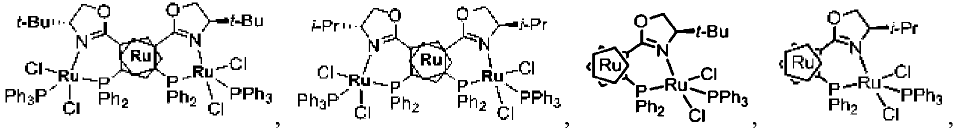 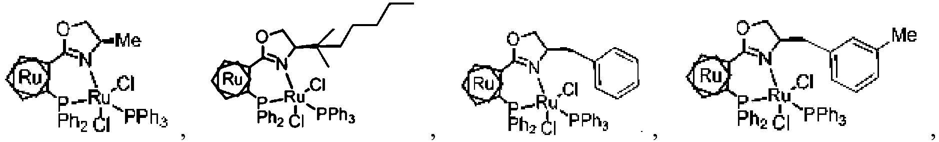 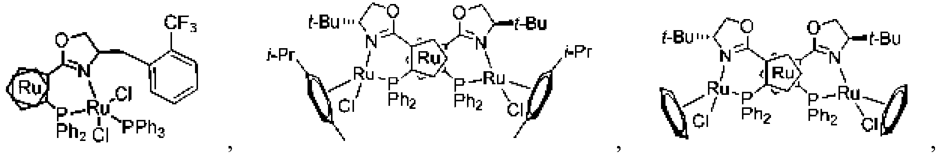 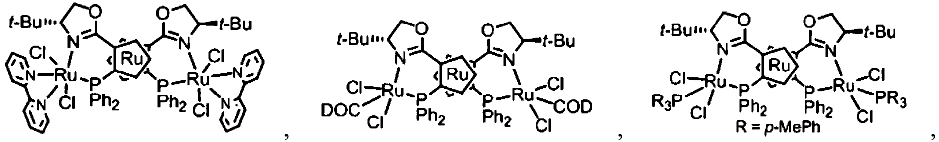 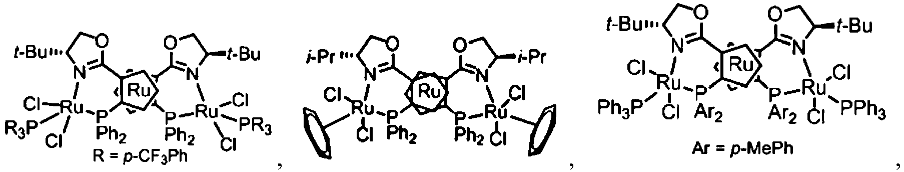 and

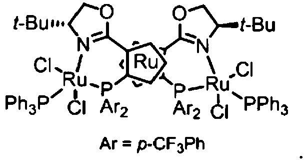

6. The asymmetric catalytic hydrogenation synthesis method according to claim 5, wherein the catalyst is selected from the compound shown in the following structure:

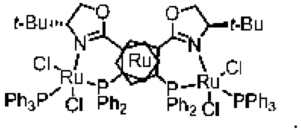

7. The asymmetric catalytic hydrogenation synthesis method according to any one of claims 1-4, wherein the solvent is selected from the group consisting of methanol, ethanol, isopropanol, propanol, butanol, isobutanol, acetone, 1,4-dioxane, tetrahydrofuran, dichloromethane, acetonitrile, toluene, xylene, and the combinations thereof, and/or
the alkali is selected from the group consisting of LiOH, NaOH, KOH, Li₂CO₃, Na₂CO₃, K₂CO₃, Cs₂CO₃, LiHCO₃, NaHCO₃, KHCO₃, CsHCO₃, Na₃PO₄, Na₂HPO₄, NaH₂PO₄, triethylamine, pyridine, 2,6-methylpyridine, 1,8-diazabicyclo[5,4,0]-7-undecene, 1,4-diaza[2.2.2]bicyclooctane, and the combinations thereof, more preferably, NaOH and/or KOH; and/or
the molar ratio of the compound represented by formula I to the alkali is 1:0.1~3.

8. The asymmetric catalytic hydrogenation synthesis method according to any one of claims 1-4, wherein the reaction temperature of the asymmetric catalytic hydrogenation synthesis method is -20~60 °C, and/or
the hydrogen pressure of the asymmetric catalytic hydrogenation synthesis method is 1 to 80 bar, and/or
the molar ratio of the substrate to the catalyst in the asymmetric catalytic hydrogenation synthesis method is 10000:1~10:1.

9. The asymmetric catalytic hydrogenation synthesis method according to claim 8, wherein the reaction temperature of the asymmetric catalytic hydrogenation synthesis method is 15-50 °C, and/or
the hydrogen pressure of the asymmetric catalytic hydrogenation synthesis method is 40 to 60 bar, and/or
the molar ratio of the substrate to the catalyst in the asymmetric catalytic hydrogenation synthesis method is 3000:1~100:1.

10. The asymmetric catalytic hydrogenation synthesis method according to any one of claims 1-4, wherein the method for generating a catalyst by reacting a planar chiral metallocene ligand with a ruthenium salt comprises the steps of: reacting a planar chiral metallocene ligand with a ruthenium salt in a solvent at 60-120 °C to generate a catalyst,
preferably, the solvent used for the reaction of a planar chiral metallocene ligand with a ruthenium salt is selected from the group consisting of methanol, ethanol, isopropanol, propanol, butanol, isobutanol, acetone, 1,4-dioxane, tetrahydrofuran, dichloromethane, acetonitrile, toluene, xylene, and the combinations thereof.

11. The asymmetric catalytic hydrogenation synthesis method according to claim 10, wherein the asymmetric catalytic hydrogenation synthesis method comprises the following steps:
(1) reacting a planar chiral ruthenocene ligand with a ruthenium salt in a solvent, and after the reaction is completed, treating and separating the reaction solution to obtain a catalyst;
(2) adding the compound shown in formula I, the catalyst obtained in step (1), an alkali, and a solvent into a reaction vessel, and conducting the asymmetric hydrogenation reduction in a hydrogen atmosphere to form the compound shown in formula II,
the solvents used in step (1) and step (2) may be the same or different.

12. The asymmetric catalytic hydrogenation synthesis method according to claim 10, wherein the asymmetric catalytic hydrogenation synthesis method comprises the following steps:
(1) reacting a planar chiral ferrocene ligand with a ruthenium salt in a solvent to form a catalyst, obtaining a reaction solution containing the catalyst,
(2) adding the compound of formula I, an alkali, and a solvent to the reaction solution containing the catalyst in step (1), in a hydrogen atmosphere, the compound shown in formula I undergoing the asymmetric hydrogenation reduction to form the compound shown in formula II, the solvents used in step (1) and step (2) are preferably the same.

13. A synthesis method for brivaracetam, wherein the synthesis method comprises the following steps:
(1) subjecting the compound shown in formula II obtained by the asymmetric catalytic hydrogenation synthesis method according to any one of claims 1 to 12 to esterification reaction to synthesize the compound shown in formula II-1,
(2) subjecting the compound shown in II-1 to an amination reaction to synthesize brivaracetam, and the reaction formula is as follows: 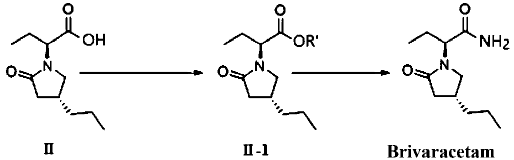 in formula II-1, R' is selected from aliphatic or aromatic hydrocarbon groups.

14. The synthesis method according to claim 11, wherein the esterification reaction of step (1) is carried out in methanol or ethanol solvent in the presence of thionyl chloride, and/or
the amination reaction of step (2) is carried out in a mixed solvent of ammonia water with a mass concentration of 15-28% and alcohol, under the condition of introduced ammonia gas at -10-5 °C, wherein the alcohol is methanol and/or ethanol.

15. A use of the compound as shown in the following structure for asymmetric catalytic hydrogenation of 1,5-dihydropyrrol-2-one and the derivatives thereof, wherein the 1,5-dihydropyrrol-2-one and the derivatives thereof undergo hydrogenation reduction to generate 2-pyrrolidone and the derivatives thereof,

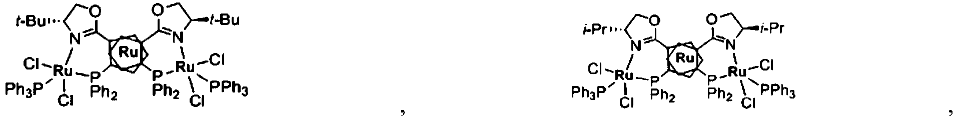

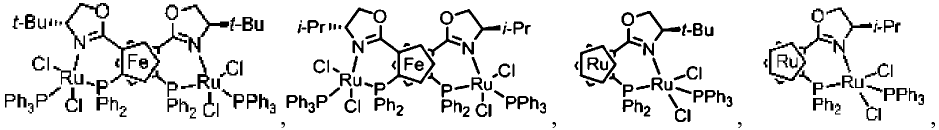

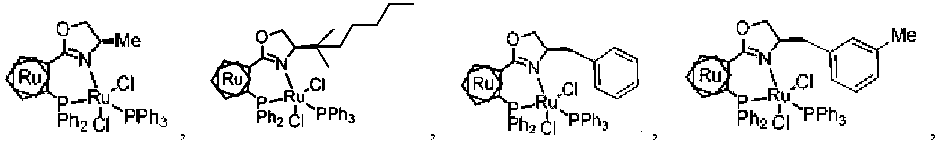

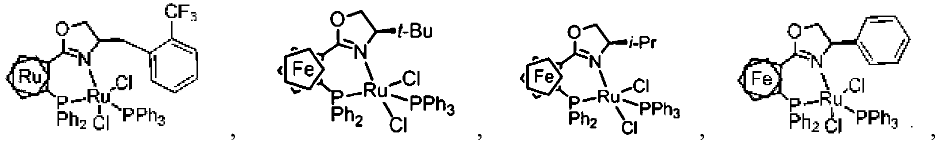

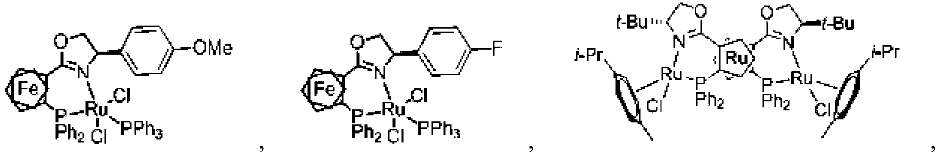

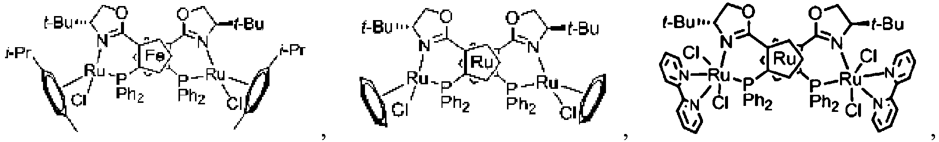

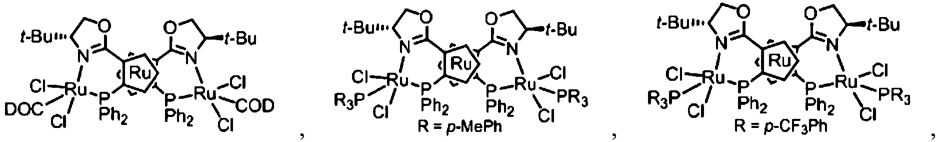

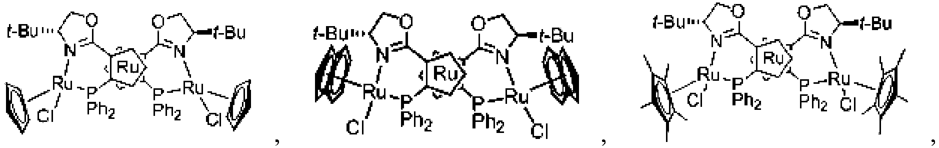

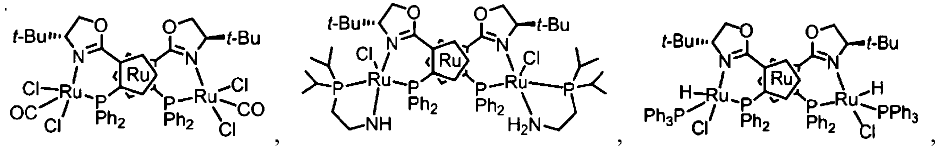

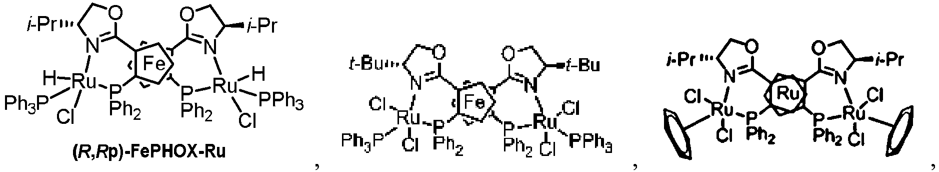

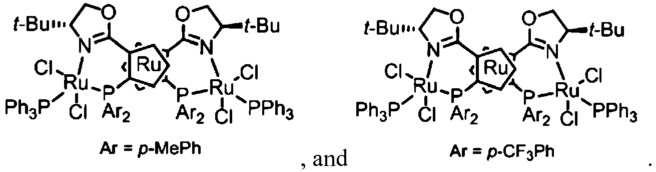

16. A use of the compound as shown in the following structure for asymmetric catalytic hydrogenation of 1,5-dihydropyrrol-2-one and the derivatives thereof, wherein 1,5-dihydropyrrol-2-one and the derivatives thereof undergo hydrogenation reduction to generate 2-pyrrolidone and the derivatives thereof,

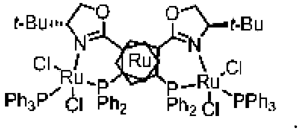

17. A catalyst for asymmetric hydrogenation reduction, which has the following structure: 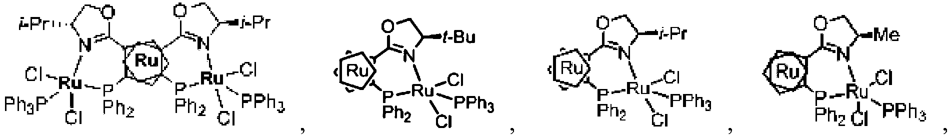 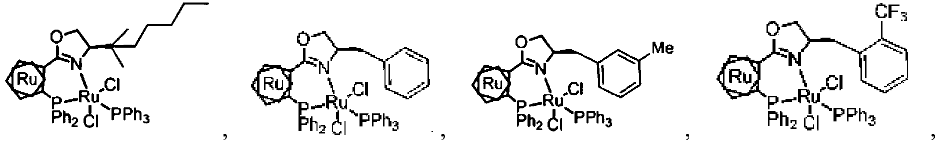 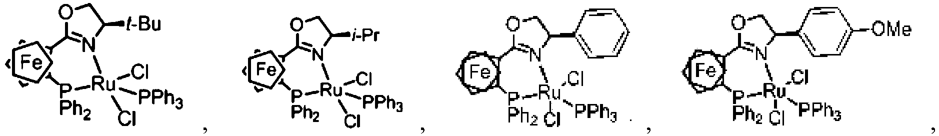 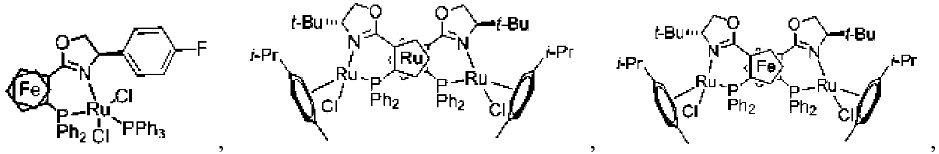 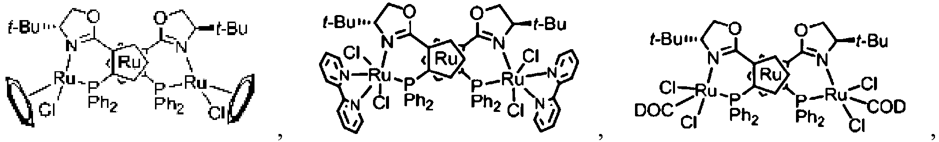 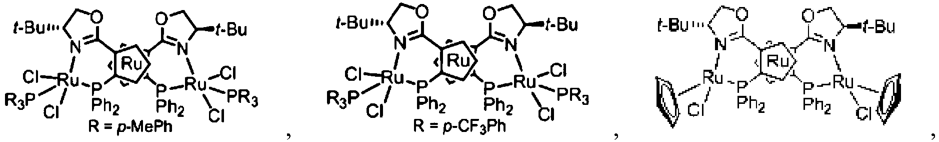 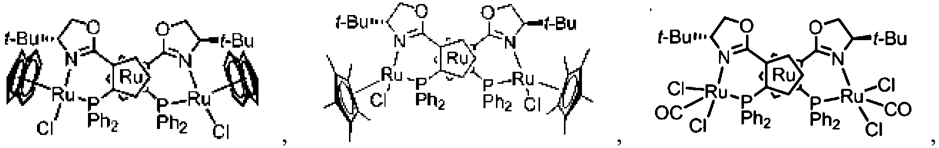 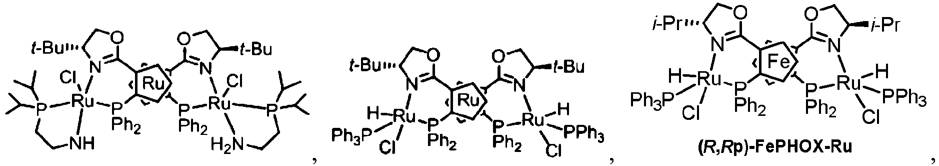 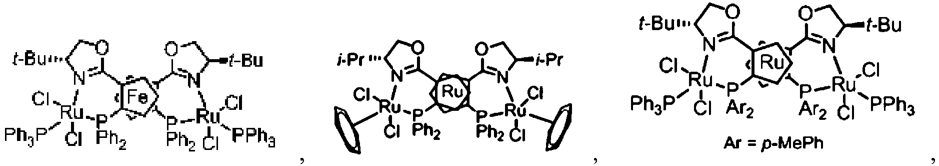 and
